# EUROPÄISCHE PATENTANMELDUNG

(11) **EP 4 345 094 A1**
(43) Veröffentlichungstag der Anmeldung: **03.04.2024**
(21) Anmeldenummer: 22199231.6
(22) Anmeldetag: 30.09.2022
(51) Int. Cl.: C07C 271/06, C01B 32/80

(54) **VERFAHREN ZUR PHOSGEN-HERSTELLUNG MIT RÜCKFÜHRUNG VON KOHLENDIOXID AUS WERTSTOFFRECYCLING**

(71) Anmelder: Covestro Deutschland AG, 51373 Leverkusen (DE)
(72) Erfinder: Die Erfindernennung liegt noch nicht vor
(74) Vertreter: Levpat

(57) **Zusammenfassung**

Die Erfindung betrifft ein Verfahren zur Herstellung von Phosgen, insbesondere für die Umsetzung von Phosgen mit organischer Aminoverbindung zu organischem Isocyanat. In diesem Verfahren wird zumindest ein aus einer Hydrolyse von organisch modifiziertem Carbamat gebildeter und gegebenenfalls von Nebenbestandteilen aufgereinigter Kohlendioxid Gasstrom reduktiv zu Kohlenmonoxid umgesetzt. Das resultierende Kohlenmonoxid gelangt in einer Phosgensynthese zur Umsetzung mit Chlor zu Phosgen. Das organisch modifizierte Carbamat für das Verfahren lässt sich bevorzugt aus einer Chemolyse von end-of-life Polyurethan-Material, insbesondere mit Alkoholen, Aminen oder Aminoalkoholen als Chemolysereagenz, gewinnen.

## Beschreibung

Die Erfindung betrifft ein Verfahren zur Herstellung von Phosgen, insbesondere für die Umsetzung von Phosgen mit organischer Aminoverbindung zu organischem Isocyanat, worin zumindest ein aus einer Hydrolyse von organischem Carbamat gebildeter und gegebenenfalls von Nebenbestandteilen aufgereinigter Kohlendioxid Gasstrom zu Kohlenmonoxid umgesetzt wird, und das resultierende Kohlenmonoxid in einer Phosgensynthese mit Chlor zu Phosgen umgesetzt wird.

Die Rückführung von Kohlendioxid (im Weiteren auch als CO₂ bezeichnet) in die Wertstoffkette stellt einen Beitrag zur Nachhaltigkeit dar. In der Vergangenheit gab es einige Bestrebungen, CO₂ beispielsweise für die Herstellung von Kohlenmonoxid (Letzteres im Weiteren auch als CO bezeichnet) nutzbar zu machen.

In der Druckschrift EP 3 744 812 B1 wird beschrieben, das bei der Pyrolyse von Polyurethan-Wertstoff gebildetes CO₂ sowie bei der Verbrennung von Polyurethan-Wertstoff gebildetes CO₂ in einer COz-Elektrolyse unter Reduktion des CO₂ zu CO umgesetzt wird. Das erhaltene CO wird mit Chlor zu Phosgen umgesetzt, welches der Herstellung von organischen Isocyanaten als Baustein von Polyurethan dient. Insbesondere das bei der Verbrennung erhaltene CO₂ muss einem aufwendigen Reinigungsverfahren unterworfen werden, bevor es in einer elektrochemischen CO₂-Reduktion genutzt werden kann.

Der Rückführungsprozess von CO₂ in die Produktion von Isocyanatverbindungen gemäß Druckschrift WO 2021/089737 A1 sieht vor, CO₂ aus einer externen Quelle, wie beispielsweise einer Verbrennung von Polyurethan-Wertstoff, in einer reverse watergas shift reaktion (RWGS Reaktion) mit Wasserstoff zu CO umzusetzen. Auch hier muss das CO₂ vor dem Einsatz in der RWGS Reaktion aufwendig gereinigt werden.

Eine weitere Möglichkeit CO₂ in die Produktion von Isocyanatverbindungen zurückzuführen bietet der Prozess gemäß Druckschrift WO 2022/167387 A1. Hierin wird das in einem Dampfreformierungs-Prozess gebildete CO₂ sowie zusätzlich CO₂ aus einer weiteren CO₂-Quelle in den Dampfreformierungs-Prozess wieder zurückgeführt. Als weitere CO₂-Quelle wird beispielsweise das CO₂, das bei einer Zementherstellung oder bei einer Verbrennung von Abfällen anfällt, genannt. CO₂ aus diesen Quellen muss gleichfalls vor dem Einsatz im Dampfreformierungs-Prozess aufwendig gereinigt werden.

Die mit vorliegender Erfindung gelöste Aufgabe ist es, den Vefahrensaufwand der CO₂ Verwertungsprozesse des Standes der Technik zu reduzieren, um die Prozesse ökonomischer und/oder ökologischer zu gestalten. Insgesamt soll ein nachhaltiges Verfahren der Herstellung und Wiederverwertung von Polyurethan-Material bereitgestellt werden.

Unter "Nachhaltigkeit" eines Verfahrens versteht der Fachmann in Anwendung der durch die UN geprägte Nachhatigkeits-Definition ("sustainable development") gemäß Brundtlandbericht der "Weltkommission für Umwelt und Entwicklung", dass durch die Ausführung des Verfahrens in der Gegenwart ein möglichst geringer bis gar kein Beitrag geleistet wird, dass künftige Generationen der Menschheit ihre eigenen Bedürfnisse nicht mehr befriedigen können, insbesondere Bedürfnisse mit Blick auf die Nutzung von Ressourcen wie z.B. fossiler Rohstoffe und insbesondere mit Blick auf die Schonung des Lebensraumes, wie z.B. den Schutz der Erdatmosphäre.

Es wurde gefunden, dass das bei der Hydrolyse von organisch modifizierten Carbamat-Verbindungen gebildete CO₂ (insbesondere das bei der Hydrolyse von einem organisch modifizierten und aus einer Chemolyse von Polyurethan-Material erhaltenem Carbamat gebildete CO₂) einen sehr hohen Reinheitsgrad aufweist und mit geringerem Einsatz ökonomischer und/oder ökologischer Ressourcen für die reduktive Umsetzung zu CO bereitgestellt werden kann.

Ein erster Gegenstand der Erfindung ist somit ein Verfahren zur Herstellung von Phosgen (insbesondere für die Herstellung von organischem Isocyanat) durch zumindest folgende Schritte
Herstellung eines CO₂ Gasstroms durch zumindest die folgenden Schritte:
Bereitstellung mindestes eines organisch modifizierten Carbamats;
Hydrolyse des bereitgestellten mindestens einen organisch modifizierten Carbamats, zumindest unter Bildung von organischer Aminoverbindung und CO₂;
Abtrennung des gebildeten CO₂ unter Erhalt mindestens eines CO₂ Gasstroms;
bevorzugt Reinigung des CO₂ Gasstroms von Nebenbestandteilen (insbesondere von Nebenbestandteilen ausgewählt aus mindestens einer Verbindung aus der Liste, die gebildet wird aus Alkoholen, Polyethern, Kohlenwasserstoffen, organischen Aminen, Wasser, Schwefelverbindungen, Staub, Sauerstoff und Stickstoff) mittels mindestens einer Reinigungsmethode ausgewählt aus Kondensation, Adsorption, katalytischer Gasreinigung oder Gaswäsche unter Erhalt eines gereinigten Kohlendioxids;
Reduktion von CO₂ des mindestens einen hergestellten, gegebenenfalls gereinigten CO₂ Gasstroms zu Kohlenmonoxid,
Synthese von Phosgen zumindest aus dem durch besagte Reduktion erhaltenen Kohlenmonoxid und Chlor.

Aus den Druckschriften DE 197 19 084 A1, EP 0 031 538 A2, WO 2022/171586 A1 und WO 2022/128871 A1 ist beispielsweise bekannt, dass durch eine Chemolyse von Polyurethan-Wertstoff organische Amine und Polyole als solche chemische Verbindungen aus dem Polyurethan-Wertstoff gewonnen werden können, die für die Herstellung von Polyurethan-Material wiederverwertbar sind.

Ein Ansatz zum stofflichen Recycling von Polyurethan-Material ist hierbei z.B. eine Alkoholyse oder Glykolyse, bei der die Urethangruppe mit Alkohol bzw. Glykol durch Umesterung bzw. Umurethanisierung umgesetzt wird:

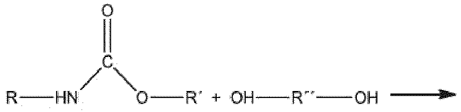

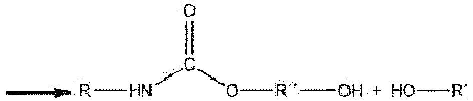

Weiterhin kann die Urethangruppe mit einem Amin unter Bildung einer Ureylen-Gruppe umgesetzt werden:

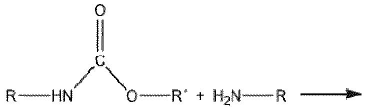

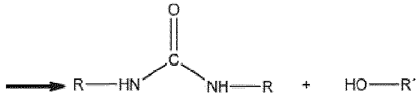

Die besondere Eignung von CO₂-haltigem Abgas aus der Hydrolyse organisch modifizierter Carbamate, beziehungsweise aus dem Chemolyse-Prozess von Polyurethan-Material, für eine Wiederverwertung wird nirgends beschrieben.

"Polyurethan-Material" im Sinne der vorliegenden Erfindung sind die Polyadditionsprodukte (bisweilen, wenn auch nicht ganz korrekt, auch als Polykondensationsprodukte bezeichnet), die durch Umsetzung von mehrwertigen *Isocyanaten* (= Iscocyanatkomponente der Polyurethanherstellung) mit *Polyolen* (= Polyolkomponente der Polyurethanherstellung) erhalten werden. Polyurethan-Material enthält im Allgemeinen neben der oben skizzierten Polyurethangrundstruktur noch andere Strukturen, zum Beispiel Strukturen mit Harnstoffbindungen. Das Vorliegen derartiger von der reinen Polyurethangrundstruktur abweichender Strukturen neben Polyurethanstrukturen verlässt den Rahmen der vorliegenden Erfindung nicht. Polyurethan-Material sind insbesondere Polyurethan*schäume*, erhalten durch Umsetzung von mehrwertigen Isocyanaten mit Polyolen in Gegenwart eines Treibmittels.

In der Terminologie der vorliegenden Erfindung umfasst der Begriff *Isocyanate* alle dem Fachmann im Zusammenhang mit der Polyurethanchemie bekannten Isocyanate wie insbesondere Toluylendiisocyanat (TDI; hergestellt aus Toluylendiamin, TDA), die Di- und Polyisocyanate der Diphenylmethanreihe (MDI; hergestellt aus den Di- und Polyaminen der Diphenylmethanreihe, MDA), 1,5-Pentandiisocyanat (PDI; hergestellt aus 1,5-Pentandiamin, PDA), 1,6-Hexamethylen¬diisocyanat (HDI; hergestellt aus 1,6-Hexamethylendiamin, HDA), Isophorondiisocyanat (IPDI; hergestellt aus Isophorondiamin, IPDA) und Xylylendiisocyanat (XDI; hergestellt aus Xylylendiamin, XDA). Der Ausdruck *"ein Isocyanat"* umfasst selbstverständlich auch Ausführungsformen, in denen zwei oder mehr unterschiedliche Isocyanate (z. B. Gemische aus MDI und TDI) in der Herstellung des Polyurethanschaums eingesetzt wurden, es sei denn, etwas anderes wird ausdrücklich zum Ausdruck gebracht, etwa durch die Formulierung *"genau ein Isocyanat".* Die Gesamtheit aller in der Herstellung des Polyurethanschaums eingesetzten Isocyanate wird als *Isocyanatkomponente* (des Polyurethanschaums) bezeichnet. Die Isocyanatkomponente umfasst mindestens ein Isocyanat. Analog wird Gesamtheit aller in der Herstellung des Polyurethanschaums eingesetzten Polyole als *Polyolkomponente* (des Polyurethanschaums) bezeichnet. Die Polyolkomponente umfasst mindestens ein Polyol.

In der Terminologie der vorliegenden Erfindung umfasst der Begriff *Polyole* alle dem Fachmann im Zusammenhang mit der Polyurethanchemie bekannten Polyole wie insbesondere Polyetherpolyole, Polyesterpolyole, Polyetherester-Polyole und Polyethercarbonatpolyole. Der Ausdruck *"ein Polyol"* umfasst selbstverständlich auch Ausführungsformen, in denen zwei oder mehr unterschiedliche Polyole in der Herstellung des Polyurethanschaums eingesetzt wurden. Wird daher im Folgenden beispielsweise von *"einem Polyetherpolyol"* (oder *"einem Polyesterpolyol"* usw.) gesprochen, so umfasst diese Terminologie selbstverständlich auch Ausführungsformen, in denen zwei oder mehr verschiedene Polyetherpolyole (oder zwei oder mehr verschiedene Polyesterpolyole usw.) in der Herstellung des Polyurethan-Materials eingesetzt wurden.

Als "organisch modifiziertes Carbamat" werden in der Terminologie der vorliegenden Erfindung organische Urethanverbindungen bezeichnet. Darunter fallen sowohl Polyurethan-Material als auch bevorzugt diejenigen organisch modifizierten Carbamate, die bei der Chemolyse von Polyurethan-Material durch Umsetzung mit einem Chemolysereagenz gebildet werden.

Eine "organische Verbindung" enthält mindestens eine kovalente Kohlenstoff-Wasserstoff-Bindung im Molekül. Eine organische Aminoverbindung ist demnach ein organischer Stoff, der als chemische Verbindung mindestens eine Aminogruppe und zusätzlich mindestens eine kovalente Kohlenstoff-Wasserstoff-Bindung im Molekül enthält. Ein organisch modifiziertes Carbamat ist *mutatis mutandis* definiert.

Unter einer "Chemolyse" wird erfindungsgemäß die chemische Umwandlung von Polyurethan-Material unter Spaltung der Polyurethan-Polymerstruktur durch Umesterung (auch als Umurethanisierung bezeichnet) oder durch Bildung einer Ureylengruppe (auch: Carbonyldiimino-Gruppe, *-NH-C(=O)-NH-*) verstanden.

Als "Chemolysereagenz" wird ein Reagenz verstanden, das mit der Urethanbindung als reaktive Gruppe des Polyurethan-Materials eine chemische Reaktion zur Durchführung einer Chemolyse eingeht.

Im Rahmen einer bevorzugten Ausführungsform des Verfahrens wird das organisch modifizierte Carbamat durch eine Chemolyse von Polyurethan-Material bereitgestellt. Für die Bereitstellung des organisch modifizierten Carbamats ist es im Rahmen dieser Ausführungsform ausreichend, wenn das organisch modifizierte Carbamat ein unmittelbares Verfahrensprodukt einer Chemolyse von Polyurethan-Material ist. Dies bedeutet, dass es bei für die Ausführung des Schrittes der Bereitstellung des organisch modifizierten Carbamats bereits ausreicht, das besagte organisch modifizierte Carbamat lediglich im Rahmen einer Belieferung als Rohstoff aus einem Lagerbehälter oder aus einer Zuleitung zu entnehmen. In diesem Falle führt der Phosgen-Produzent als Ausführender des erfindungsgemäßen Verfahrens die Chemolyse des Polyurethan-Materials zur Herstellung des organisch modifizierte Carbamats nicht selbst aus, sondern er sorgt nur dafür, dass das bereitgestellte organisch modifizierte Carbamat durch einen Lieferanten entsprechend durch Chemolyse hergestellt wurde und dessen Verfahrensprodukt ist. Eine bevorzugte Ausführungsform des Verfahrens ist somit dadurch gekennzeichnet, dass als bereitgestelltes organisch modifiziertes Carbamat mindestens ein unmittelbares Verfahrensprodukt einer Chemolyse von Polyurethan-Material eingesetzt wird, umfassend mindestens folgenden Verfahrensschritte:
Bereitstellung von Polyurethan-Material;
Umsetzung des bereitgestellten Polyurethan-Materials mit mindestens einem Chemolysereagenz (insbesondere ausgewählt aus (a) einem primären oder sekundären organischen Amin, (b) einem Aminoalkohol mit einer primären oder sekundären Aminogruppe oder (c) einem Alkohol mit mindestens einer Hydroxylgruppe), unter Bildung von mindestens einem organisch modifizierten Carbamat.

Bei dem bereitgestellten Polyurethan-Material kann es sich grundsätzlich um jede Art von Polyurethanprodukt handeln, d. h. sowohl um Polyurethanschäume als auch um Polyurethanprodukte aus den sog. CASE-Anwendungen. Bei den Polyurethanschäumen kommen sowohl Weichschäume als auch Hartschäume in Frage, wobei Weichschäume (beispielsweise aus alten Matratzen, Polstermöbeln oder Autositzen) bevorzugt sind. Bei Polyurethanprodukten aus den CASE-Anwendungen sind Polyurethanelastomere, Polyurethanklebstoffe und Polyurethanbeschichtungen bevorzugt. Von allen Polyurethanprodukten sind Polyurethan-Weichschäume besonders bevorzugt. Das bereitgestellte Polyurethan-Material wird beispielsweise aus Polyurethan-Material Abfall von entsprechenden end-of-life Polyurethanprodukten (wie z.B. Matratzen, Polstern oder Isolationsmaterialien) bereitgestellt.

Weiterhin ist solches Polyurethan-Material bevorzugt, das hinsichtlich der Isocyanatkomponente auf einem Isocyanat ausgewählt aus der Gruppe bestehend aus Toluylendiisocyanat (TDI), den Di- und Polyisocyanaten der Diphenylmethanreihe (MDI), 1,5-Pentandiisocyanat (PDI), 1,6-Hexamethylendiisocyanat (HDI), Isophorondiisocyanat (IPDI) und Xylylendiisocyanat (XDI) und Mischungen von zwei oder mehr der vorgenannten Isocyanate basiert. Besonders bevorzugt ist Polyurethan-Material, das hinsichtlich der Isocyanatkomponente auf TDI oder einer Mischung aus TDI und MDI basiert. Ganz besonders bevorzugt ist Polyurethan-Material, das hinsichtlich der Isocyanatkomponente nur auf TDI basiert.

Weiterhin ist solches Polyurethan-Material bevorzugt, das hinsichtlich der Polyolkomponente auf einem Polyol ausgewählt aus der Gruppe bestehend aus einem Polyetherpolyol, einem Polyesterpolyol, einem Polyetherester-Polyol, einem Polyethercarbonatpolyol und einer Mischung von zwei oder mehr der vorgenannten Polyole basiert.

Bevorzugt umfasst die Bereitstellung des Polyurethan-Materials vorbereitende Schritte für die nachfolgende Umsetzung mit dem Chemolysereagenz. Dabei handelt es sich insbesondere um ein mechanisches Zerkleinern des Polyurethan-Materials. Solche vorbereitenden Schritte sind dem Fachmann bekannt; es sei beispielsweise auf den Übersichtsartikel von Simón, Borreguero, Lucas und Rodriguez in Waste Management 2018, 76, 147 - 171 verwiesen.

Es hat sich als besonders bevorzugt herausgestellt, wenn das bereitgestellte Polyurethan-Material einer Inertisierung unterzogen wurde, mindestens umfassend eine Entfernung des gasförmigen Sauerstoffs, der das Polyurethan-Material umgibt und/oder in dessen Poren eingelagert ist. Die Durchführung einer Interisierung empfiehlt sich insbesondere, wenn das Polyurethan-Material in Form eines Pulvers, eines Granulats oder eines Schaums vorliegt. Durch eine Inertisierung des bereitgestellten Polyurethan-Materials vor der Umsetzung mit dem Chemolysereagenz wird eine besonders hohe Reinheit des bei der Hydrolyse erhaltenen CO₂-Gasstroms erzielt. Sollte beispielsweise bei einem Polyurethan-Material in Form eines Schaumstoffes nicht inertisiert werden, weist das bei der Hydrolyse erhaltene CO₂ eine erhöhte Konzentration an N₂ und insbesondere an O₂ auf, das mit hohem Aufwand aus dem CO₂ Gasstrom entfernt werden muss.

Die Inertisierung umfasst in einer bevorzugten Ausführungsform zusätzlich einen Schritt der Zuführung mindestens eines Inertgases zu dem Polyurethan-Material (insbesondere von Stickstoff, Kohlendioxid, Argon oder Helium, ganz besonders bevorzugt von Kohlendioxid)

Eine Inertisierung des bereitgestellten Polyurethan-Materials wird besonders bevorzugt durch mindestens folgende Schritte durchgeführt:
Entgasung des Polyurethan-Materials, wobei in einem Behälter ein erster Druck von maximal 960 mbar_{(abs.)} und einer Temperatur von maximal 120 °C angelegt wird und das Gas der überstehenden Gasphase über eine Gasabführeinrichtung abgeführt wird. Zur Abführung des Gases wird in dem Behälter bevorzugt als erster Druck ein Unterdruck von weniger als 700 mbar_{(abs.)}, bevorzugt ein Druck von 0,1 mbar_{(abs.)} bis 100 mbar_{(abs.),} angelegt und das Gas über die Gasabführeinrichtung angeführt.

Nach der Abführung des Gases wird mindestens ein Inertgas (insbesondere Stickstoff, Kohlendioxid, Argon oder Helium, ganz besonders bevorzugt Kohlendioxid) zur Einstellung eines Drucks von bevorzugt maximal 1,8 bar_{(abs.)} , besonders bevorzugt von Atmosphärendruck, zugeführt.

Eine Durchführung der Entgasung sowie der gesamten Inertisierung wird in der Druckschrift WO 2022/128871 A1 beschrieben, auf die hier ausdrücklich und vollinhaltlich Bezug genommen wird.

Ebenso ist es im Rahmen einer weiteren Ausführungsform der Erfindung möglich, wenn zur Bereitstellung des organisch modifizierten Carbamats vorgenannte Chemolyse-Umsetzung als integraler Schritt des Verfahrens zur Herstellung von Phosgen durch den Phosgen-Produzenten selbst ausgeführt wird und danach das dabei erhaltene organisch modifizierte Carbamat weiter der Hydrolyse unter Bildung von CO₂ zugeführt wird. Daher kennzeichnet sich eine entsprechende bevorzugte Ausführungsform des Verfahrens dadurch, dass der besagte CO₂ Gasstrom (31) durch Chemolyse von Polyurethan-Material hergestellt wird, umfassend mindestens die Schritte
Bereitstellung von Polyurethan-Material;
Umsetzung des bereitgestellten Polyurethan-Materials mit mindestens einem Chemolysereagenz (insbesondere ausgewählt aus (a) einem primären oder sekundären organischen Amin, (b) einem Aminoalkohol mit einer primären oder sekundären Aminogruppe oder (c) einem Alkohol mit mindestens einer Hydroxylgruppe), unter Bildung von mindestens einem organisch modifiziertem Carbamat;
Hydrolyse des zuvor gebildeten organisch modifizierten Carbamats unter Bildung zumindest von organischer Aminoverbindung und CO₂;
Abtrennung des gebildeten CO₂ unter Erhalt mindestens eines CO₂ Gasstroms.

Im Rahmen der vorgenannten Ausführungsformen der Erfindung kann der Hydrolyseschritt als ein von der Umsetzung des Polyurethan-Materials separater Verfahrensschritt erfolgen und/oder gleichzeitig in einem Schritt. Bei einer gleichzeitigen Abfolge wird ein Gemisch, enthaltend Chemolysereagenz und zusätzlich ausreichend Wasser verwendet.

Generell ist es bevorzugt, wenn das bereitgestellte organisch modifizierte Carbamat durch eine Umsetzung des bereitgestellten Polyurethan-Materials mit mindestens einem Chemolysereaganz unter Sauerstoffausschluss gebildet wird beziehungsweise ein unmittelbares Verfahrensprodukt einer solchen Umsetzung ist. Dies bedeutet, dass die Umsetzung in einer Inertgasatmosphäre (insbesondere in einer Stickstoff-, Kohlendioxid-, Argon- oder Heliumatmosphäre, besonders bevorzugt einer Kohlendioxidatmosphäre) durchgeführt wird. Bevorzugt werden auch die eingesetzten Chemolysereagenzien (Wasser und Chemolysereagenz) durch Inertgassättigung von Sauerstoff befreit. In diesem Zusammenhanf ist es ganz besonders bevorzugt, wenn das bereitgestellte Polyurethan-Material wie oben beschrieben inertisiert wird.

Die Bereitstellung von organisch modifiziertem Carbamat wird im Rahmen einer bevorzugten Ausführungsform durch mindestens folgende Verfahrensschritte erzielt:
Bereitstellung von Polyurethan-Material;
Umsetzung des bereitgestellten Polyurethan-Materials mit mindestens einem Chemolysereagenz (insbesondere ausgewählt aus (a) einem primären oder sekundären organischen Amin, (b) einem Aminoalkohol mit einer primären oder sekundären Aminogruppe oder (c) einem Alkohol mit mindestens einer Hydroxylgruppe), unter Bildung einer Zusammensetzung, enthaltend mindestens ein organisch modifiziertes Carbamat;
Vermengen der zuvor erhaltenen Zusammensetzung ohne vorherige Abtrennung gegebenenfalls in dieser Zusammensetzung vorhandenen Wassers mit einem organischen Lösungsmittel, das mit dem zuvor eingesetzten Chemolysereagenz nicht vollständig mischbar ist, und Phasentrennung in eine Carbamatphase (enthaltend organisch modifiziertes Carbamat) und eine Lösungsmittelphase.

Die dabei erhaltene Carbamatphase wird im Rahmen dieser Ausführungsform in den Schritt der Hydrolyse eingebracht.

Das zuvor erwähnte, einzusetzende organische Lösungsmittel soll mit dem in der Chemolyse eingesetzten Chemolysereagenz nicht vollständig mischbar sein. Dies bedeutet, dass es unter den für obige Vermengung vorliegenden Bedingungen eine Mischungslücke derart geben muss, dass eine Phasentrennung möglich wird. Das organische Lösungsmittel wird besonders bevorzugt ausgewählt aus der Gruppe bestehend aus aliphatischen Kohlenwasserstoffen, alicyclischen Kohlenwasserstoffen, aromatischen Kohlenwasserstoffen und Mischungen von zwei oder mehr der vorgenannten organischen Lösungsmittel.

Im Rahmen der Integration der Chemolyse als Verfahrensschritt im Rahmen einer weiteren, bevorzugten Ausführungsform der erfindungsgemäßen Phosgenherstellung ist es besonders bevorzugt, wenn die besagte Umsetzung von Polyurethan-Material zu besagtem organisch modifiziertem Carbamat und die besagte Hydrolyse des organisch modifizierten Carbamats gemeinsam in einem Verfahrensschritt verwirklicht werden.

Im Allgemeinen werden Wasser für die Hydrolyse und Chemolysereagenz für die chemolytische Umsetzung im Rahmen des erfindungsgemäßen Verfahrens bevorzugt überstöchiometrisch eingesetzt, egal, ob die chemolytische Umsetzung allein oder in einem Schritt in Kombination mit der Hydrolyse stattfindet. Dies bedeutet, dass Wasser für die Hydrolyse in einer solchen Menge eingesetzt wird, die theoretisch ausreicht, sämtliche Polyurethanbindungen des bereitgestellten Polyurethan-Materials unter Freisetzung zumindest des Kohlenstoffdioxids und von organischer Aminoverbindung zu hydrolysieren. In gleicher Weise bedeutet der überstöchiometrische Einsatz von Chemolysereagenz, dass dieses in einer solchen Menge eingesetzt wird, die theoretisch ausreicht, sämtliche Polyurethanbindungen des bereitgestellten Polyurethan-Materials unter Bildung von organisch modifizierten Carbamaten umzusetzen. Bei Einsatz der nachfolgend beschriebenen erfindungsgemäß bevorzugten Massenanteile von Wasser und Chemolysereagenz ist Beides regelmäßig der Fall.

Bevorzugt die chemolytische Umsetzung so durchgeführt, dass das Massenverhältnis von (insgesamt eingesetztem) Chemolysereagenz und (insgesamt eingesetztem) Wasser einerseits zu dem bereitgestellten Polyurethan-Material andererseits (also [m(Chamolysereagenz) + m(Wasser)] / m(Polyurethan-produkt), m = Masse) im Bereich von 0,5 bis 2,5, besonders bevorzugt von 1,0 bis 1,3, liegt, wobei die Masse des Wassers 2,0 % bis 10 % der Masse des Chemolysereagenzes beträgt. Die quantitativen Angaben in Bezug auf Wasser beziehen sich hierbei auf das als Reagenz für die Hydrolyse zugegebene Wasser. Im Vergleich dazu sind evtl. ohnehin vorhandene Wassermengen aus Feuchtigkeit im eingesetzten Chemolysereagenz und/oder im eingesetzten Polyurethan-Material gering. Mit Feuchtigkeit im eingesetzten Chemolysereagenz bzw. im eingesetzten Polyurethan-Material sind Feuchtigkeitsspuren gemeint, wie sie im industriellen Maßstab auftreten können. Die Masse des Wassers beträgt hierbei weiter bevorzugt 4,0 % bis 10,0 %, besonders bevorzugt 5,0 % bis 7,0 %, von der Masse des eingesetzten Chemolysereagenzes.

Die chemolytische Umsetzung und die Hydrolyse lassen sich beispielsweise als Chemolyse des Polyurethan-Materials mit einem Chemolysereagenz und Wasser (bevorzugt in Gegenwart eines Katalysators) bei einer Temperatur im Bereich von 130 °C bis 220 °C durchführen, wobei das Massenverhältnis von Chemolysereagenz und Wasser einerseits zu dem Polyurethan-Material andererseits im Bereich von 0,5 bis 2,5 liegt und die Masse des Wassers 4,0 % bis 10 % der Masse des Polyurethan-Materials beträgt.

Im Rahmen der gemeinsamen Verwirklichung der Chemolyse und Hydrolyse in einem Verfahrensschritt hat es sich als besonders bevorzugt herausgestellt, das einzusetzende Wasser für die Hydrolyse nicht gleich zu Beginn des Reaktionszeitraums zuzugeben, zumindest nicht vollständig zuzugeben. Es hat sich bewährt, zunächst gar kein oder nur einen geringen Anteil, nämlich 2 % bis 4 %, der insgesamt einzusetzenden Menge an Wasser mit den übrigen Reaktanden (i.e. zumindest Chemolysereagenz und Polyurethan-Material) zu vermischen und die restliche bzw. gesamte Menge an Wasser im weiteren Verlauf über die Reaktionsdauer zuzugeben. Die Zugabe des Wassers für die Hydrolyse der als Intermediat gebildeten organisch modifizierten Carbamate erfolgt dabei kontinuierlich oder portionsweise in Intervallen in der Weise, dass die Siedetemperatur des Reaktionsgemisches stets in den spezifizierten Bereichen, insbesondere in dem besonders bevorzugten Bereich von 165 °C bis 185 °C, bleibt. Die Dosierzeit des Wassers liegt bevorzugt im Bereich von 1,0 Stunden bis 5,0 Stunden (abhängig vom Siedepunkt des eingesetzten Chemolysereagenzes). In einer bevorzugten Ausführungsform wird ein Massenverhältnis von (insgesamt eingesetztem) Chemolysereagenz und (insgesamt eingesetztem) Wasser einerseits zu Polyurethan-Material andererseits [m(Chemolysereagenz + Wasser)/m(Polyurethan-Material)] im Bereich von 1,1 bis 1,3 eingesetzt. Wird das Wasser nicht auf einmal, sondern nach und nach zugegeben wie zuvor beschrieben, gilt dies für die insgesamt eingesetzte Menge an Wasser.

Die Menge an Wasser, die im Rahmen der gemeinsamen Verwirklichung der Chemolyse und Hydrolyse in einem Verfahrensschritt eingesetzt wird, beträgt bevorzugt 5,0 % bis 7,0 % der Masse des eingesetzten Chemolysereagenzes; dies gilt insbesondere in Verbindung mit dem zuvor genannten Bereich von 1,0 bis 1,3 für das Massenverhältnis [m(Chemolysereagenz + Wasser)/m(Polyurethan-Material)]. Wird das Wasser nicht auf einmal, sondern nach und nach zugegeben wie zuvor beschrieben, gilt dies für die insgesamt in diesem Schritt eingesetzte Menge an Wasser.

Im Rahmen der gemeinsamen Verwirklichung der Chemolyse und Hydrolyse in einem Verfahrensschritt ist es nicht erforderlich, sämtliches Wasser bereits zu Beginn zuzugeben. In diesem Fall beziehen sich die oben genannten Mengenbereiche des Wassers bezogen auf die Masse des Chemolysereagenzes auf die Menge Wassers, die bis zum Ende der Reaktionsdauer insgesamt zugegeben wird. Wird das Chemolysereagenz sukzessive zugegeben, gilt Entsprechendes.

Es ist insbesondere auch möglich, das Polyurethan-Material
(I) zunächst nur mit dem Chemlysereagenz oder dem Chemolysereagenz und einem ersten Teil des Wassers zu versetzen, und dann
(II) Wasser bzw. einen zweiten Teil des Wassers zuzugeben, und zwar insbesondere erst nachdem das Polyurethan-Material in Lösung gegangen ist.

Der Ausdruck "in Lösung gegangen" impliziert in diesem Zusammenhang nicht notwendigerweise das Vorliegen einer "echten" Lösung im Sinne einer vollkommen homogenen Mischung. Es kann durchaus vorkommen, dass es sich um eine trübe "Lösung" des Polyurethan-Materials handelt; derartiges verlässt den Rahmen der vorliegenden Erfindung nicht.

Im Rahmen der Durchführung der gemeinsamen Verwirklichung der Chemolyse und Hydrolyse in den vorgenannten Schritten (I) und (II) ist es insbesondere bevorzugt, in Schritt (II) das Wasser bzw. den zweiten Teil des Wassers kontinuierlich oder portionsweise so zuzugeben, dass die Temperatur der flüssigen Phase während des Schritts (II) um maximal 20 °C, bevorzugt um maximal 15 °C, besonders bevorzugt um maximal 10 °C, ganz besonders bevorzugt um maximal 5,0 °C und außerordentlich ganz besonders bevorzugt um maximal 1,0 °C von der Temperatur der flüssigen Phase aus Schritt (I) abweicht. Auf diese Weise wird erreicht, dass die Temperatur stets hoch genug ist, um ein Fortschreiten der chemolytischen Umsetzung zu gewährleisten.

Eine Durchführung einer Chemolyse in Kombination mit einer Hydrolyse in einem Schritt wird beispielsweise in der Druckschrift WO 2022/171586 A1 am Beispiel eines Alkohols mit mindestens einer Hydroxylgruppe als Chemolysereagenz beschrieben. Auf diese Druckschrift wird hier ausdrücklich und vollinhaltlich Bezug genommen.

Eine Durchführung einer Chemolyse mit einer Hydrolyse als separatem Schritt wird beispielsweise in den Druckschriften US 4,336,406 und WO 2020/260387 A1 jeweils am Beispiel eines Alkohols mit mindestens einer Hydroxylgruppe als Chemolysereagenz beschrieben. Auf diese Druckschrift wird hier ausdrücklich und vollinhaltlich Bezug genommen.

Die Chemolyse kann generell, gegebenenfalls in Kombination mit der Hydrolyse, in einem beliebigen, in der Fachwelt für einen derartigen Zweck bekannten Reaktor durchgeführt werden. Insbesondere eignen sich Rührkessel (gerührte Reaktoren) und Rohrreaktoren als Chemolysereaktoren.

Im Rahmen einer weiteren Ausführungsform ist es besonders bevorzugt, die Chemolyse als Umsetzung von Polyurethan-Material mit Chemolysereagenz (beispielsweise allein oder in einem Schritt in Kombination mit der Hydrolyse) durch den Einsatz mindestens eines Katalysators zu unterstützen. Als Katalysator wird im Rahmen dieser bevorzugten Ausführungsform besonders bevorzugt mindestens eine Verbindung eingesetzt, ausgewählt aus Carbonat, Hydrogencarbonat, Orthophosphat, Mono-Hydrogen-Orthophosphat, Metaphosphat, Hydroxid, organisches Amin (insbesondere Diethanolamin), organometallische Verbindungen (insbesondere Titantetrabutanolat, Zinnoctanoat oder Dibutylzinndilaurat) oder Mischungen von zwei oder mehr der vorgenannten Katalysatoren. Ganz besonders bevorzugt wird mindestens ein Metallsalz ausgewählt aus einem Carbonat, einem Hydrogencarbonat, einem Orthophosphat, einem Mono-Hydrogen-Orthophosphat, einem Metaphosphat oder einer Mischung von zwei oder mehr der vorgenannten Metallsalze als Katalysator eingesetzt.

Orthophosphate sind die Salze der Orthophosphorsäure, H₃PO₄, bei denen alle Protonen abgespalten wurden (= PO₄³⁻). Mono-Hydrogen-Orthophosphate sind die Salze der Orthophosphorsäure, bei denen zwei Protonen abgespalten wurden (= HPO₄²⁻). Metaphosphate sind Kondensationsprodukte der Orthophosphorsäure mit der Summenformel [(PO₃)⁻]ₙ, worin n eine natürliche Zahl (insbesondere 3 oder 4) bezeichnet.

Falls ein Katalysator in einem Chemolyseschritt eingesetzt wird, wird dieser vorzugsweise in einer solchen Menge zugesetzt, dass seine Masse 0,1 % bis 3,5 % von der Masse des bereitgestellten Polyurethan-Materials beträgt.

Wird ein Katalysator als wässrige Lösung eingesetzt, so ist das als Lösungsmittel eingesetzte Wasser ebenso bei den oben genannten quantitativen Angaben des Wassereinsatzes bei der Hydrolyse zu berücksichtigen, d. h. die erforderlichenfalls zusätzlich bei der Hydrolyse einzusetzende Wassermenge ist entsprechend zu verringern.

Es ist weiter bevorzugt, auch für die Hydrolyse mindestens einen der vorgenannten Katalysatoren einzusetzen.

Im Rahmen einer weiteren Ausführungsform ist es besonders bevorzugt, die Chemolyse (beispielsweise allein oder bevorzugt in einem Schritt in Kombination mit der Hydrolyse) als Umsetzung von Polyurethan-Material mit mindestens einem Chemolysereagenz auszuführen, ausgewählt aus (a) einem primären oder sekundären organischen Amin, (b) einem Aminoalkohol mit einer primären oder sekundären Aminogruppe oder (c) einem Alkohol mit mindestens einer Hydroxylgruppe.

Als Alkohol mit mindestens einer Hydroxylgruppe eignet sich als Chemolysereagenz bevorzugt mindestens ein Alkohol mit mindestens zwei Hydroxylgruppen. Hierbei ist es wiederum besonders bevorzugt, wenn das Chemolysereagenz ausgewählt wird aus Ethylenglykol, Diethylenglykol, Propylenglykol, Dipropylenglykol, Methylglykol, Triethylenglykol, Glycerin, 2 Methyl-1,3-Propandiol oder Mischungen von zwei oder mehr der vorgenannten Alkohole.

Als Aminoalkohol mit einer primären oder sekundären Aminogruppe eignet sich als Chemolysereagenz bevorzugt mindestens ein Aminoalkohol, ausgewählt aus Ethanolamin, N-Methylethanolamin, 3-Amino-1-propanol.

Als primäres oder sekundäres organisches Amin eignet sich als Chemolysereagenz bevorzugt mindestens ein aliphatisches primäres oder aliphatisches sekundäres organisches Amin. Bei den primären oder sekundären Aminen handelt es sich dabei vorzugsweise um Mono- und/oder Diamine. 1,2-Ethylendiamin, 1,4-Diaminobutan, 1,6-Hexamethylendiamin oder eine Mischung aus zwei oder mehr derselben sind als primäres oder sekundäres organisches Amin besonders bevorzugte Chemolysereagenzien.

Besagte chemolytische Umsetzung von Polyurethan-Material mit Chemolysereagenz zu besagtem organisch modifiziertem Carbamat wird bevorzugt bei einer Temperatur in einem Bereich von 140 °C bis 220 °C, bevorzugt 170 °C bis 200 °C durchgeführt. Diese Temperatur soll ebeso bevorzugt auch bei einer gleichzeitigen Umsetzung von Polyurethan-Material mit der Hydrolyse in einem Schritt eingehalten werden. Hinsichtlich des Drucks bestehen keine besonderen Ansprüche. Die Reaktion kann sowohl bei erniedrigtem also auch bei erhöhtem Druck durchgeführt werden; beispielsweise bei einem Druck von 200 mbar(abs.) bis 2000 mbar(abs.), bevorzugt 500 mbar(abs.) bis 1500 mbar(abs.), besonders bevorzugt 900 mbar(abs.) bis 1300 mbar(abs.) und insbesondere bei Umgebungsdruck. Druckangaben erfolgen im Rahmen der vorliegenden Erfindung stets als absolute Drücke, gekennzeichnet durch ein der Druckeinheit nachgestelltes tiefgestelltes "abs." (bspw. wird ein absoluter Druck von 900 mbar als "900 mbar_{(abs.)}" angegeben).

Gemäß einer weiteren Ausführungsform sind solche Verfahren bevorzugt, bei denen der erhaltene CO₂ Gasstrom eine Temperatur von 140 °C bis 220 °C, bevorzugt von 170 °C bis 200 °C, aufweist.

Eine Reinigung des erhaltenen Kohlendioxids von Nebenbestandteilen, insbesondere von Stickstoff, Sauerstoff, Chemolysereagenz (wie z.B. Diethylenglykol), Schwefelverbindungen, Staub und Wasser kann bevorzugt mittels mindestens einer Reinigungsmethode, ausgewählt aus Kondensation, Adsorption, katalytischer Gasreinigung oder Gaswäsche unter Erhalt eines gereinigten Kohlendioxids (31a) erfolgen. Der durch das erfindungsgemäße Verfahren erhaltene CO₂ Gasstrom weist jedoch einen hervorragenden Reinheitsgrad für die weitere Nutzung in der Reduktion von CO₂ auf. Gegebenenfalls vor der Reduktion von CO₂ sind zur Aufarbeitung des CO₂ wenige Reinigungsschritte erforderlich. Diese sind leicht und effektiv durchzuführen. Das aus der Hydrolyse erhaltene CO₂ des CO₂ Gasstroms enthält als überwiegende Nebenbestandteile Chemolysereagenz, sowie bei der Chemolyse gebildete organische Aminoverbindung und Polyol. Diese Nebenbestandteile lassen sich effektiv durch eine Reinigung (4) des CO₂ Gasstroms (31) entfernen, in der zumindest der CO₂ Gasstrom über mehrere Kondensatoren geführt und abgekühlt wird, wobei die im Gasstrom als Nebenbestandteil enthaltenen organischen und wässrigen Komponenten kondensieren, als Flüssigkeit abgetrennt werden und nach Abschluss der Reinigung (4) ein gereinigter CO₂ Gasstrom (31a) der Reduktion (6) zu Kohlenstoffmonoxid zugeführt wird.

Eine weiter verbesserte Reinigung des CO₂ Gasstroms wird erzielt, wenn eine Kombination aus Kondensation und Gaswäsche durchgeführt wird.

Das für die Synthese von Phosgen benötigte Kohlenmonoxid wird durch Reduktion (6) von CO₂ des mindestens einen, wie zuvor beschrieben hergestellten, gegebenenfalls gereinigten CO₂ Gasstroms (31, 31a) zu Kohlenmonoxid (21a) dargestellt. In einer weiteren bevorzugten Ausführungsform des Verfahrens wird die Reduktion von CO₂ zumindest durch mindestens ein Verfahren verwirklicht, ausgewählt aus
A) der elektrochemischen Reduktion von CO₂, bevorzugt unter Einsatz von aus regenerativer Energie hergestellter elektrischer Energie,
B) einem Reformerprozess, worin Methan (bevorzugt Bio-Methan) und Wasserdampf unter Zusatz von mindestens CO₂, unter Zuführung von Wärmeenergie bei einer Temperatur von mindestens 500°C zu Kohlenmonoxid umgesetzt werden,
C) Umsetzung von Wasserstoff und CO₂ in einer reverse water-gas shift RWGS-Reaktionszone zu Kohlenmonoxid.

Unter "regenerativer Energie" versteht der Fachmann Energie aus einer Energiequelle, die sich nicht erschöpft, wie z.B. Windenergie, Wasserenergie, Bioenergie (z.B. Verstromung von Biogas oder Biomasse) oder Sonnenenergie. Als regenerative Energie eignen sich daher ganz besonders bevorzugt wahlweise Windkraft, Sonnenenergie, Wasserkraft oder Mischungen daraus.

Im Rahmen der Ausführung einer Reduktion von CO₂ ist es bevorzugt, wenn der gegebenenfalls gereinigte CO₂ Gasstrom für die Reduktion (6) in eine Elektrolysevorrichtung eingebracht wird und an einer Elektrode, bevorzugt an einer Gasdiffusionselektrode, (besonders bevorzugt unter Einsatz von aus regenerativer Energie hergestellter elektrischer Energie) zu Kohlenstoffmonoxid reduziert wird.

Die CO₂-Elektrolyse kann beispielsweise eine Hochtemperaturelektrolyse sein, die bei einer Temperatur von mehr als 600°C ggf. auch unter Zusatz von Wasser zur Herstellung von Synthesegas, betrieben wird. Hochtemperaturelektrolysen sind grundsätzlich bekannt und im Markt verfügbar z.B. von Haldor Topsoe, eCOs^{®}. Bei der Hochtemperaturelektrolyse entsteht an der Anode Sauerstoff. Nachteil der bekannten Hochtemperaturelektrolyse ist ihre schlechte Scaleup Fähigkeit, so dass bei größeren CO-Mengen, z.B. mehr als 1 t/h CO, derzeit noch die Niedertemperaturelektrolyse zu bevorzugen ist.

Wird die COz-Elektrolyse als Niedertemperaturelektrolyse betrieben, so erfolgt die Elektrolyse bei einer Temperatur unter 150°C.

Bei allen CO₂ -Elektrolysen wird das CO₂ Gas dem Kathodenraum zugeführt.

Im Falle der Niedertemperaturelektrolyse wird CO₂ insbesondere an einer Gasdiffusionselektrode zu Kohlenmonoxid und ggf. Wasserstoff umgesetzt. Gleichzeitig kann an der Anode O₂ oder ggf. alternativ auch Chlor erzeugt werden. Wird an der Anode Chlor erzeugt, so kann dieses Chlor der Phosgensynthese und somit der Isocyanat-Herstellung als weiterer Rohstoff zugeführt werden. Der Fachmann kennt beispielsweise aus der Druckschrift WO 2021/069470 A Elektroden und eine Methode zur Ausführung einer elektrochemischen Reduktion von CO₂. Bevorzugterweise wird die elektrochemischen Reduktion von CO₂ nach einem Verfahren der WO 2021/069470 A durchgeführt. Auf diese Druckschrift wird hierin ausdrücklich und vollinhaltlich Bezug genommen. In diesem bevorzugten elektrolytischen Verfahren zu Herstellung von Kohlenmonoxid werden Kohlenmonoxid, ggf. Wasserstoff und Chlor durch elektrochemische Umsetzung von Kohlendioxid und Alkalichloridlösung erhalten. Dieses bevorzugte Elektrolyseverfahren ist dadurch gekennzeichnet, dass das Kohlendioxid an einer Gasdiffusionselektrode als Kathode in einer wässrigen Alkalichlorid-haltigen Lösung als Katholyt elektrochemisch reduziert und gleichzeitig Chlor aus einer wässrigen Alkalichlorid-haltigen Lösung als Anolyt anodisch erzeugt wird, wobei das im Katholyt gebildete Alkalisalz der Kohlensäure, ausgewählt aus Alkalicarbonat, Alkalihydrogencarbonat oder Mischungen daraus anschließend mit Chlorwasserstoff zu Kohlendioxid und Alkalichlorid umgesetzt wird und das hierbei freigesetzte Kohlendioxid in den Kathodenraum zur Gasdiffusionselektrode und das erzeugte Alkalichlorid wahlweise in den Anodenraum und/oder in den Kathodenraum zurückgeführt werden.

Nach den bekannten Prinzipien kann bei der Niedertemperatur-Elektrolyse auch ein MEA (Membran-Electrode-Assembly) Konzept zum Einsatz kommen. Hierbei wird auf die Membran ein Katalysator aufgebracht. Eine davor gelagerte Gasdiffusionsschicht reguliert den Gas- und Flüssigkeitstransport. Dies kann sowohl auf der Anoden- als auch auf der Kathodenseite erfolgen. Weiterhin besteht die Möglichkeit eine Gasdiffusionselektrode in direktem Kontakt mit der Membran zu bringen.

Die eingesetzte Gasdiffusionselektrode kann in einer zero-gap als auch in einer finite-gap Anordnung in der Elektrolysezelle verbaut sein. Eine bevorzugte Anordnung für eine Niedertemperaturelektrolyse von CO₂ wird in der Druckschrift WO 2020/057998 A1 beschrieben, auf die ausdrücklich und vollinhaltlich Bezug genommen wird.

Dem Kathodenraum, bzw. der darin installierten Gasdiffusionselektrode, kann ein Überschuss an CO₂ zugeführt werden. Überschuss bedeutet, mehr CO₂ einzuleiten als für die stöchiometrische Umsetzung aufgrund des fließenden elektrischen Stromes notwendig wäre. Somit gelangt aus dem Kathodenraum eine Gasmischung bestehend aus nicht umgesetzten CO₂, CO und H₂.

Ein geeigneter Reformerprozess, worin Methan (bevorzugt Bio-Methan) und Wasserdampf unter Zusatz von mindestens besagtem CO₂, unter Zuführung von Wärmeenergie bei einer Temperatur von mindestens 500°C zu Kohlenmonoxid umgesetzt werden, wird in der PCT-Patentanmeldung mit der Anmeldenummer PCT/EP2022/052267 beschrieben, auf die ausdrücklich und vollinhaltlich Bezug genommen wird. Dieses Verfahren betrifft die Herstellung von Kohlenmonoxid aus Methan, Wasserdampf und CO₂ für die Darstellung von Phosgen zur Synthese von organischem Isocyanat, enthaltend zumindest die Schritte

Synthese von Kohlenmonoxid in einem Reformerprozess, worin Methan und Wasserdampf unter Zusatz von mindestens CO₂ unter Zuführung von Wärmeenergie bei einer Temperatur von mindestens 500°C zu Kohlenmonoxid-haltigem Produktgas umgesetzt wird,

Reinigung des aus vorgenannter Synthese erhaltenen Kohlenmonoxid-haltigen Produktgases, mindestens durch Abtrennung von CO₂ und gegebenenfalls zusätzlich durch mindestens eine Abtrennung ausgewählt aus der Abtrennung von Wasser, der Abtrennung von Wasserstoff oder einer Kombination davon, unter Erhalt von Kohlenmonoxid;

Bereitstellung von CO₂ für den besagten Zusatz zum vorgenannten Reformerprozess zumindest aus besagter Abtrennung von CO₂ des vorgenannten Reinigungsschritts,
mit der Maßgabe, dass
1) die zur Synthese von Kohlenmonoxid dem Reformerprozess zugeführte Wärmeenergie durch mindestens eine Methode bereitgestellt wird, ausgewählt aus (i) der Verbrennung von Brennstoff, der mittels regenerativer Energie erzeugten Wasserstoff enthält, (ii) der Verbrennung von Brennstoff, der Methan aus biologischer Quelle enthält, (iii) Umwandlung von aus regenerativer Energie erzeugter, elektrischer Energie in Wärme; oder
2) für die Bereitgestellung des der Synthese des vorgenannten Reformerprozesses zugesetzten CO₂ zusätzlich CO₂ aus einer weiteren CO₂-Quelle herangezogen wird; oder
3) eine Kombination der vorgenannten Möglichkeiten 1) und 2) gewählt wird.

Als Quelle von Methan kommt für den Reformerprozess besonders bevorzugt Methan aus biologischer Quelle in Frage. Unter "Methan aus biologischer Quelle" (auch als Bio-Methan" bezeichnet, versteht der Fachmann in Abgrenzung zu fossilem Methan solches Methan, welches technisch durch Methan-Gärung aus Biomasse gewonnen wird. Die Methan-Gärung ist bekanntermaßen der anaerobe Abbau organischer Stoffe durch Mikroorganismen. Methan aus biologischer Quelle wird beispielsweise in Biogasanlagen hergestellt, worin sowohl organische Abfälle als auch nachwachsende Rohstoffe entsprechend vergoren werden.

Als eine weitere CO₂-Quelle dient für den oben genannten Schritt 2) bevorzugt mindestens eine externe CO₂-Quelle, die CO₂ beiträgt, welches nicht durch das erfindungsgemäße Verfahren emittiert wird. Eine externe CO₂ Quelle wäre z.B. das CO₂, das bei einer Zementherstellung, bei einer H₂-Herstellung für die Ammoniaksynthese anfällt, bei Verbrennung von Brennstoffen (z.B. einer Abfallverbrennung) im Abgas entsteht, oder CO₂, das aus der Luft gewonnenen wird. Dieses CO₂ aus einer externen CO₂-Quelle wird im Rahmen einer bevorzugten Ausführungsform des erfindungsgemäßen Verfahrens durch Absorption eines CO₂ -Anteils aus (i) Prozessgasen oder Abgasen, die aus mindestens einem Prozess ausgewählt aus Zementherstellung, H₂-Herstellung, Verbrennung ausgewählt wird und/oder (ii) aus Luft durch einleiten in Alkalilauge, zum Beispiel Kalilauge, erfolgen. Hierbei bildet sich Kaliumhydrogencarbonat, welches anschließend wieder zu CO₂ und Kalilauge thermisch zersetzt werden kann. Das dabei freigesetzte CO₂ wird dann dem Reformerprozess zur Synthese von Kohlenmonoxid zugeführt. Solches freigesetztes CO₂ kann auch unabhängig von der Durchführung eines Reformerprozesses zusätzlich einer Reduktion von CO₂ gemäß A) (elektrochemische Reduktion) oder C) (RWGS Reaktion) zugeführt werden.

Im Rahmen einer besonders bevorzugten Ausführungsform des erfindungsgemäßen Verfahrens wird das Kohlenmonoxid für die Phosgensynthese, insbesondere für die Synthese von Phosgen, aus besagtem CO₂ hergestellt, durch mindestens eine Umsetzung von Wasserstoff und CO₂ in einer reverse water-gas shift (RWGS) Reaktionszone zu Kohlenmonoxid. Ein solche Ausführungsform wird beispielsweise in dem Dokument WO 2021/089737 A beschrieben, auf das hierin ausdrücklich und vollinhaltlich Bezug genommen wird. Eine erfindungsgemäß ganz besonders bevorzugte Ausführungsform des Verfahrens ist dadurch gekennzeichnet, dass ein Wasserstoffstrom bereitgestellt wird und zusammen mit dem gegebenenfalls gereinigten CO₂ Gasstrom nach dem Prinzip der reverse water gas shift-Reaktion (insbesondere in einer Reaktionszone) zu einem Produktgas, enthaltend Kohlenstoffmonoxid und gegebenenfalls Nebenprodukte umgesetzt wird.

Eine "Reaktionszone" ist der Teil eines Reaktionsraumes, in dem eine chemische Reaktion, z.B. die reverse watergas shift Reaktion, abläuft. Ein "Reaktionsraum" ist ein Volumen, in dem die an einer chemischen Reaktion beteiligten Reaktionspartner zusammengebracht werden und in dem die chemische Reaktion stattfindet. Dies kann für eine chemische Reaktion beispielsweise das Volumen eines Gefäßes sein, in dem sich ein Edukt, z.B. im Falle einer RWGS-Reaktion Kohlendioxid, und dessen Reaktionspartner, im Falle einer RWGS-Reaktion Wasserstoff, gemeinsam befinden und in der Reaktionszone zur Reaktion gebracht werden.

In diesem besonders bevorzugten Verfahren werden zur Kohlenmonoxid-Herstellung ganz besonders bevorzugt mindestens folgende Schritte durchgeführt:
Einspeisung von bereitgestelltem Wasserstoffgas zusammen mit dem besagten CO₂ Gasstrom in eine RWGS Reaktionszone und Umsetzung der Edukte nach dem Prinzip der RWGS Reaktion zu einem Produktgasgemisch aus Wasserdampf, CO und gegebenenfalls Nebenprodukten, insbesondere niedere Kohlenwasserstoffe, insbesondere bevorzugt Methan;
Abtrennung von nicht umgesetztem Kohlendioxid aus dem aus der Abtrennung erhaltenen Gasgemischs der RWGS Reaktion, insbesondere mittels Aminwäsche, und Rückführung des nicht umgesetzten Kohlendioxids in die RWGS Reaktion;
Abtrennung des in der RWGS Reaktion nicht umgesetzten Wasserstoffs aus dem nach der Abtrennung erhaltenen Gasgemisch von Kohlenmonoxid und Wasserstoff, insbesondere unter Verwendung einer Coldbox, und wahlweise Rückführung des Wasserstoffs in die RWGS Reaktion;
Einspeisung des verbliebenen Kohlenmonoxids aus der Abtrennung in die Synthese von Phosgen.

Die RWGS Reaktion wird bevorzugt in Gegenwart mindestens eines Katalysators durchgeführt. Dieser ist besonders bevorzugt ausgewählt aus mindestens einer Verbindung aus der Gruppe:
(I) Mischmetalloxide der Formel A_{(1-w-x)}A'_{w}A"ₓB_{(1-y-z)}B'_{y}B"_{z}O_{3-delta}
   wobei hier gilt:
   A, A' und A" sind unabhängig voneinander ausgewählt aus der Gruppe: Mg, Ca, Sr, Ba, Li, Na, K, Rb, Cs, Sn, Sc, Y, La, Ce, Pr, Nd, Pm, Sm, Eu, Gd, Tb, Dy, Ho, Er, Tm. Yb, Tl, Lu, Ni, Co, Pb, Bi und/oder Cd; und
   B, B' und B" sind unabhängig voneinander ausgewählt aus der Gruppe: Cr, Mn, Fe, Bi, Cd, Co, Cu, Ni, Sn. AI, Ga, Sc, Ti, V, Nb, Ta, Mo, Pb. Hf, Zr, Tb. W, Gd. Yb, Mg, Li, Na, K, Ce und/oder Zn; und
   0 ≤ w ≤ 0.5; 0 ≤ x < 0.5; 0 ≤ y ≤ 0.5; 0 ≤ z ≤ 0.5 und - 1 ≤ delta ≤ I;
(II) Mischmetalloxide der Formel A_{(1-w-x)}A'_{w}A"ₓB_{(1-y-z)}B'_{y}B"_{z}O_{3-delta} wobei hier gilt:
   A, A' und A" sind unabhängig voneinander ausgewählt aus der Gruppe: Mg, Ca, Sr, Ba. Li, Na, K, Rb, Cs, Sn, Sc, Y, La, Ce, Pr, Nd, Sm, Eu, Gd, Tb, Dy, Ho, Er, Tm, Yb, Tl, Lu, Ni, Co, Pb und/oder Cd; und
   B ist ausgewählt aus der Gruppe: Cr, Mn, Fe, Bi, Cd, Co, Cu, Ni, Sn, AI, Ga, Sc, Ti, V, Nb, Ta, Mo, Pb, Hf, Zr, Tb, W, Gd, Yb, Mg, Cd, Zn, Re, Ru, Rh, Pd, Os, Ir und/oder Pt; und
   B' ist ausgewählt aus der Gruppe: Re, Ru, Rh, Pd, Os, Ir und/oder Pt; und B" ist ausgewählt aus der Gruppe: Cr, Mn, Fe, Bi, Cd, Co, Cu, Ni, Sn, AI, Ga, Sc, Ti, V, Nb, Ta, Mo, Pb, Hf, Zr, Tb. W. Gd, Yb. Mg, Cd und/oder Zn; und
   0 ≤ w ≤ 0,5; 0 ≤ x ≤ 0,5; 0 ≤ y ≤ 0,5; 0 ≤ z ≤ 0.5 und - I ≤ delta ≤ 1 ;
(III) Mischungen von wenigstens zwei verschiedenen Metallen M1 und M2 auf einem Träger, welcher ein mit einem Metall M3 dotiertes Oxid von AI, Ce und/oder Zr umfasst;
   wobei hier gilt: M1 und M2 sind unabhängig voneinander ausgewählt aus der Gruppe: Re, Ru, Rh, Ir, Os, Pd und/oder Pt; und
   M3 ist ausgewählt aus der Gruppe: Sc, Y, La, Ce, Pr, Nd, Sm, Eu, Gd, Tb, Dy, Ho, Er, Tm, Yb und/oder Lu;
(IV) Mischmetalloxide der Formel LOₓ(M_{(y/z)}Al_{(2-y/z)}O₃)_{z}; wobei hier gilt:
   L ist ausgewählt aus der Gruppe: Na, K, Rb, Cs, Mg, Ca, Sr, Ba, Sc, Y, Sn, Pb, Pd, Mn, In, Tl, La, Ce, Pr, Nd, Sm, Eu, Gd, Tb, Dy, Ho, Er, Tm. Yb und/oder Lu; und
   M ist ausgewählt aus der Gruppe: Ti, Zr, Hf, V, Nb, Ta, Cr, Mo, W, Mn, Re, Fe, Ru, Os, Co, Rh. Ir, Ni, Pd. Pt. Zn, Cu, Ag und/oder Au; und
   1 < x ≤ 2; 0 < y ≤ 12; und 4 ≤ z ≤ 9;
(V) Mischmetalloxide der Formel LO(Al₂O₃)_{z}; wobei hier gilt:
   L ist ausgewählt aus der Gruppe: Na, K, Rb. Cs, Mg, Ca, Sr, Ba, Sc, Y, Sn, Pb, Mn, In, Tl, La, Ce, Pr, Nd, Sm, Eu, Gd, Tb, Dy, Ho, Er, Tm, Yb und/oder Lu; und
   4 ≤ z ≤ 9;
(VI) oxidischer Katalysator, der Ni und Ru umfasst.
(VII) Metall M1 und/oder wenigstens zwei verschiedene Metalle M1 und M2 auf und/oder in einem Träger, wobei der Träger
   ein Carbid, Oxycarbid, Carbonitrid, Nitrid, Borid, Silicid, Germanid und/oder Selenid der Metalle A und/oder B ist; wobei hier gilt:
   M1 und M2 sind unabhängig voneinander ausgewählt aus der Gruppe: Cr, Mn, Fe, Co, Ni, Re, Ru, Rh, Ir, Os, Pd, Pt, Zn, Cu, La, Ce, Pr, Nd, Sm, Eu, Gd, Tb, Dy, Ho, Er, Tm, Yb, und/oder Lu; und
   A und B sind unabhängig voneinander ausgewählt aus der Gruppe: Be, Mg, Ca, Sc, I i, V, Cr, Mn, Fe, Co, Ni, Y, Zr, Nb, Mo, Hf, Ta, W, La, Ce, Pr, Nd, Sm, Eu, Gd, Tb, Dy, Ho, Er, Tm, Yb, und/oder Lu;
      und/oder
   Reaktionsprodukte von (I), (II), (III), (IV), (V), (VI) und/oder (VII) in Gegenwart von Kohlendioxid, Wasserstoff, Kohlenmonoxid und/oder Wasser bei einer Temperatur von ≥ 700 °C.

Der für die RWGS-Reaktion bereitgestellte Wasserstoffstrom wird im Rahmen einer weiteren Ausführungsform bevorzugt durch eine Elektrolyse von Wasser zu Wasserstoff und Sauerstoff erhalten. Die Wasserelektrolyse zur Herstellung von Wasserstoffgas stellt die elektrochemische Umwandlung von Wasser dar. Unter Nutzung von Strom wird hier Wasser in Wasserstoff und Sauerstoff gespalten. Bekannte Verfahren sind die Protonen-Austausch-Elektrolyse (PEM-Elektrolyse), welche bspw. in EP 3489394 A beschrieben wird. Ein weiteres Verfahren stellt die alkalische Wasserelektrolyse dar. Beide Verfahren unterscheiden sich durch insbesondere durch die Verfahrensparameter wie bspw. Temperatur, Druck und pH-Wert. Beide Verfahren führen jedoch zu den Zielprodukten Wasser- und Sauerstoffgas. Die Wasserelektrolyse kann mit Anlagen nach dem Stand der Technik durchgeführt werden. Bekannt und kommerziell erhältlich sind technische Anlagen zur alkalischen Wasserelektrolyse als auch zur PEM-Elektrolyse. Die Prinzipien der Wasserelektrolyse sind beispielhaft in Kapitel 6.3.4 in Volkmar M. Schmidt in "Elektrochemische Verfahrenstechnik" (2003 Wiley-VCH-Verlag; ISBN 3-527-29958-0) beschrieben.

Besonders bevorzugt ist es, wenn die Wasserelektrolyse (5) zur Bereitstellung des Wasserstoffstroms unter Verwendung von aus regenerativer Energie erzeugtem elektrischem Strom durchgeführt wird, insbesondere aus regenerativer Energie in Form von Windkraft, Sonnenenergie oder Wasserkraft.

Das aus der Reduktion von CO₂ erhaltene Kohlenmonoxid wird nötigenfalls von gegebenenfalls vorhandenen Nebenprodukten gereinigt und zur Synthese von Phosgen eingesetzt. Dies Synthese von Phosgen wird durch Umsetzung von zumindest Chlorgas und Kohlenmonoxid, wobei als Kohlenmonoxid mindestens das Kohlenmonoxid aus der Reduktion von CO₂ umgesetzt wird und Phosgen ausgebracht wird.

Die Reinigung des Kohlenmonoxids erfolgt bevorzugt vor dem Einsatz in der Synthese von Phosgen durch mindestes folgende Schritte:
Abtrennung von Wasser,
Abtrennung von nicht umgesetztem Kohlendioxid aus dem aus der Abtrennung von Wasser erhaltenen Gasgemischs, insbesondere mittels Aminwäsche und Rückführung des nicht umgesetzten Kohlendioxids in die Reduktion von CO₂ des erfindungsgemäßen Verfahrens,
Abtrennung von gegebenenfalls vorhandenem Wasserstoff aus dem nach der Abtrennung von nicht umgesetzten Kohlendioxid erhaltenen Gasgemisch, insbesondere unter Verwendung einer Coldbox.

Im Rahmen einer weiteren bevorzugten Ausführungsform des Verfahrens erfolgt die Abtrennung von Kohlendioxid nachdem zuvor Wasser aus dem Kohlenmonoxid-haltigen Produktgas abgetrennt wurde. Zu diesem Zweck wird das Kohlenmonoxid-haltige Gas zunächst einer Wasser-Abtrenneinheit zugeführt, dort das Wasser abgetrennt und das nach der Abtrennung von Wasser erhaltene Kohlenmonoxid-haltige, trockene Produktgas einer CO₂-Abtrenneinheit zugeführt, bei der das CO₂ abgetrennt wird. In der Wasser-Abtrenneinheit wird zur Abtrennung des Wassers beispielsweise das Kohlenmonoxid-haltige Gas abgekühlt und das Wasser, beispielsweise als Kondensat, abgetrennt.

Unter "Aminwäsche" wird hier insbesondere die grundsätzlich bekannte Wäsche nach dem Prinzip der Chemisorption mit Aminen wie Monoethanolamin (MEA) Diethanolamin (DEA), Methyldiethanolamin (MDEA) oder Diglycolamin (DGA) verstanden, welche schon bei relativ niedrigem Druck in einer Absorptionskolonne eine hohe Reinheit des gereinigten Gasgemisches erreicht.

Als erfindungsgemäß bevorzugte Variante des Verfahrens gilt ein Verfahren, in dem das (bevorzugt vorher von Wasser und von CO₂ befreite) Kohlenmonoxid-haltige Gas in eine H₂-CO Trenneinheit eingebracht wird, in der Wasserstoff abgetrennt wird. Dabei bildet sich zumindest ein Gasstrom, dessen Gas bei 25°C und 1013 mbar mindestens 95 Vol.% Kohlenmonoxid, bevorzugter mindestens 99 Vol.% Kohlenmonoxid, enthält. Das besagte eingebrachte Produktgas wird in der H₂-CO Trenneinheit vorzugsweise zunächst in zwei Gasströme aufgetrennt. Es entsteht dabei ein Gas in Form eines Gasstroms, das mindestens 95 Vol.% (bevorzugt mindestens 99 Gew.-%) Kohlenmonoxid enthält, ein weiteres Gas in Form eines Gasstroms, dessen größter Bestandteil Wasserstoff ist und unter anderem Kohlenmonoxid und Methan enthält. Das weitere Gas wird auch als Restgas der H₂-CO Trennung oder falls keine Restgasbehandlung erfolgt, als Endgas bezeichnet. Eine nach diesem Prinzip der Auftrennung arbeitende H₂-CO Trenneinheit ist die sogenannte Coldbox. Das Wasserstoff-haltige Restgas der H₂-CO Trennung kann zur Aufkonzentrierung des Wasserstoffs einer anschließenden Restgasbehandlung ausgesetzt werden. Nach der Aufarbeitung durch die Restgasbehandlung wird ein mit Wasserstoffgas angereichertes Gas in Form eines Gasstroms und ein weiteres Gas - das sogenannte Restgas der Restgasbehandlung oder Endgas - in Form eines Gasstroms, erhalten, das eine Mischung von CO, Methan und einer geringeren Menge Wasserstoff enthält, als das mit Wasserstoffgas angereicherte Gas.

Für die Bereitstellung des Chlors für die im Rahmen dieser Ausführungsform erfolgende Synthese von Phosgen ist dem Fachmann die Herstellung von Chlorgas aus elektrochemischer Oxidation nach der Salzsäure-Elektrolyse mit Gasdiffusionselektrode (auch als HCl ODC Elektrolyse-Verfahren bezeichnet (ODC steht für Oxygen Depleting Electrode, als eine ODC wird beispielsweise die sogenannte SVK (Sauerstoffverzehrkathode) eingesetzt); geeignete Elektrolysezelle vgl. US,6022,634 A, WO 03/31690 A1), die Herstellung von Chlorgas aus Salzsäure-Diaphragmaelektrolyse (vgl. EP 1 103 636 A1), die Herstellung von Chlorgas aus thermokatalytischer Gasphasenoxidation (vgl. WO 2012/025483 A2), sowie die Herstellung von Chlor aus Chloralkali-Elektrolyse (vgl. WO 2009/007366 A2) hinlänglich bekannt. Auf den Inhalt der vorgenannten, im Zusammenhang mit der Herstellung von Chlorgas zitierten Schriften wird ausdrücklich und vollumfänglich Bezug genommen. Das für die Synthese von Phosgen benötigte Chlor wird in einer bevorzugten Variante dieser Ausführungsform des Verfahrens elektrolytisch hergestellt, insbesondere durch elektrochemische Oxidation nach der Salzsäure-Elektrolyse mit Gasdiffusionselektrode, durch elektrochemische Oxidation nach der Salzsäure-Diaphragmaelektrolyse oder durch elektrochemische Oxidation nach der Chloralkali-Elektrolyse. Dabei ist es wiederum besonders bevorzugt, wenn die besagte elektrochemische Oxidation jeweils unter Verwendung von aus regenerativer Energie erzeugtem elektrischem Strom durchgeführt wird, insbesondere aus regenerativer Energie in Form von Windkraft, Sonnenenergie oder Wasserkraft.

Das durch die Phosgensynthese gebildete Phosgen wird im Rahmen einer besonders bevorzugten Ausführungsform des Verfahrens in einem weiteren Schritt zur Herstellung von organischem Isocyanat eingesetzt, worin das Phosgen aus der Phosgensynthese mit mindestens einer organischen Aminoverbindung, insbesondere mit bei der Hydrolyse des organisch modifizierten Carbamats erhaltenen organischen Aminoverbindung, umgesetzt und zumindest organisches Isocyanat ausgebracht wird. Vorzugsweise werden zumindest organisches Isocyanat und Chlorwasserstoff ausgebracht.

Es sind generell solche erfindungsgemäßen Verfahren dieser Ausführungsform bevorzugt, in denen das gewonnene organische Isocyanat mindestens zwei Isocyanatgruppen enthält. Zu diesem Zweck wird bei der Synthese wiederum bevorzugt als Reaktand organische Aminoverbindung mit mindestens zwei Aminogruppen, insbesondere die bei der Hydrolyse des organisch modifizierten Carbamats erhaltene organische Aminoverbindung, eingesetzt.

Besonders bevorzugt enthält das gewonnene organische Isocyanat mindestens zwei Isocyanatgruppen und weist eine Molmasse von höchstens 1000 g/mol, insbesondere von höchstens 800 g/mol, auf.

Ganz besonders bevorzugt eingesetzte organische Amine werden ausgewählt aus Toluylendiamin (TDA), Methylendi(phenylamin) (MDA) (wiederum bevorzugt ausgewählt aus Diphenylmethan-2,2'-diamin, Diphenylmethan-2,4'-diamin, Diphenylmethan-4,4`-diamin oder Mischungen daraus), Hexamethylendiamin, Isophorondiamin, 1,3-Bis(aminomethyl)benzol, Cyclohexyldiamin oder Mischungen daraus, wobei TDA, MDA oder Mischungen daraus, insbesondere das bei der Hydrolyse des organisch modifizierten Carbamats erhaltene TDA oder MDA, ganz besonders bevorzugte organische Isocyanate sind.

Ganz besonders bevorzugt erhaltene organische Isocyanate werden ausgewählt aus Toluylendiisocyanat (TDI), Methylendi(phenylisocyanat) (MDI) (wiederum bevorzugt ausgewählt aus Diphenylmethan-2,2'-diisocyanat, Diphenylmethan-2,4'-diisocyanat, Diphenylmethan-4,4'-diisocyanat oder Mischungen daraus), Hexamethylendiisocyanat (HDI), Isophorondiisocyanat (IPDI), 1,3-Bis(isocyanatomethyl)benzol (XDI), Cyclohexyldiisocyanat (CHDI) oder Mischungen daraus, wobei TDI, MDI oder Mischungen daraus ganz besonders bevorzugte organische Isocyanate sind.

Ferner entsteht im Rahmen der Ausführungsform mit Synthese von organischem Isocyanat neben besagtem organischen Isocyanat bevorzugt zusätzlich Chlorwasserstoff. Es ist wiederum bevorzugt diesen Chlorwasserstoff als Edukt der Herstellung von Chlor für die besagte Synthese von Phosgen zuzuführen. Die dafür notwendige Abtrennung und Reinigung des in der Isocyanat-Herstellung gebildeten Chlorwasserstoffs kann anschließend erfolgen durch eine oxidative Umsetzung in einer thermokatalytischen Gasphasenoxidation mit Sauerstoff zu Chlor und Wasser und ggf. Nutzung von O₂ aus der Wasserelektrolyse.

Eine weitere alternative Vorgehensweise bei der Nutzbarmachung des Chlorwasserstoffs als Edukt für die Herstellung von Chlor ist die Umsetzung des Chlorwasserstoffes mit Wasser zu Salzsäure und anschließender elektrochemischer Oxidation der Salzsäure zu Chlor und ggf. Wasserstoff. Diese wässrige Salzsäure wird insbesondere der zuvor beschriebenen elektrochemischen Oxidation nach der Salzsäure-Elektrolyse mit Gasdiffusionselektrode, der elektrochemischen Oxidation nach der Salzsäure-Diaphragmaelektrolyse oder der elektrochemischen Oxidation nach der Chloralkali-Elektrolyse) zugeführt. Im Falle der Salzsäure-Elektrolyse mit Gasdiffusionselektrode benötigte O₂ kann aus der Wasserelektrolyse bezogen werden.

Der anfallende Chlorwasserstoff aus der Isocyanat-Herstellung kann ebenso nach Reinigung und Absorption in Wasser zu Salzsäure umgesetzt und die resultierende Salzsäure in den Markt für diverse Anwendungen verkauft werden.

Im Rahmen einer besonders bevorzugten Ausführungsform des Verfahrens wird zusätzlich
eine Phosgensynthese durch Umsetzung von zumindest von Chlorgas und dem zuvor nach der Reinigung des besagten Kohlenmonoxid-haltigen Produktgases aus Schritt c) erhaltenem Kohlenmonoxid;
eine Isocyanat-Herstellung durch Umsetzung des erhaltenen Phosgens mit mindestens einem organischen Amin zu mindestens einer organischen Isocyanat-Verbindung und Chlorwasserstoff;
eine Abtrennung und Reinigung des in der Isocyanat-Herstellung gebildeten Chlorwasserstoffs und eine anschließende oxidative Umsetzung des Chlorwasserstoffs, ausgewählt aus
   - einer Umsetzung von Chlorwasserstoff in einer thermokatalytischen Gasphasenoxidation mit Sauerstoff zu Chlor und Wasser gegebenenfalls unter Nutzung von Sauerstoff aus der Wasserelektrolyse,
   - einer Umsetzung von Chlorwasserstoff mit Wasser zu Salzsäure und anschließender elektrochemischer Oxidation der Salzsäure zu Chlor und gegebenenfalls Wasserstoff;
eine an die oxidative Umsetzung des Chlorwasserstoffs anschließende Zuführung des gebildeten Chlors in die besagte Synthese von Phosgen, gegebenenfalls unter Zuführung einer zusätzlichen Menge Chlor in die besagte Synthese von Phosgen, die aus einer Chloralkali-Elektrolyse stammt;
durchgeführt.

Die erhaltenen organischen Isocyanate können optional in einem zusätzlichen Schritt des Verfahrens mit mindestens einer organischen Verbindung mit mindestens zwei Hydroxygruppen, insbesondere mindestens einem Polyesterpolyol oder Polyetherpolyol, zu Polyurethan-Material umgesetzt werden.

Entsprechend hergestellte Polyurethan-Materialien sind beispielsweise in Form von Schäumen, Lacken, Isoliermasse, Bestandteil von Marktprodukten. Sie können am Ende ihres Lebenszyklus wieder als Polyurethan-Material für das erfindungsgemäße Verfahrens bereitgestellt werden.

Ein weiterer Gegenstand der Erfindung ist die Verwendung von CO₂, das unmittelbares Produkt eines Verfahrens ist, enthaltend zumindest den Schritt einer Hydrolyse mindestens eines organisch modifizierten Carbamats, in einer Reduktionsreaktion (i.e. eine Reduktion von CO₂) zur Herstellung von Kohlenstoffmonoxid für die Phosgenherstellung.

Entsprechende Ausführungsformen des erfindungsgemäßen Verfahrens gelten *mutatis mutandis* auch für diesen Gegenstand der Erfindung.

Noch ein weiterer Gegenstand der Erfindung ist die Verwendung von Kohlenstoffmonoxid, das unmittelbares Produkt eines Verfahrens, enthaltend zumindest eine Reduktionsreaktion von CO₂ ist, das durch Hydrolyse mindestens einer organischen Verbindung ausgewählt aus organisch modifiziertem Carbamat hergestellt wurde, zur Herstellung von Phosgen.

Entsprechende Ausführungsformen des erfindungsgemäßen Verfahrens gelten *mutatis mutandis* auch für diesen Gegenstand der Erfindung.

Noch ein weiterer Gegenstand der Erfindung ist eine Vorrichtung zur Herstellung von Phosgen, enthaltend
mindestens eine Einheit zur Bereitstellung eines CO₂ Gasstroms, der unmittelbares Produkt eines Verfahrens ist, das zumindest den Schritt einer Hydrolyse mindestens einer organischen Verbindung ausgewählt aus organisch modifiziertem Carbamat enthält, wobei diese Einheit zumindest mindestens einen CO₂-Auslass für besagten CO₂ Gasstrom umfasst; und
gegebenenfalls mindestens eine Einheit zur Reinigung des CO₂ Gasstroms von Nebenbestandteilenenthaltend mindestens eine Einheit aus der Gruppe, die gebildet wird aus Kondensationseinheit, Adsorptionseinheit, Gaswäscheeinheit oder katalytischer Gasreinigungseinheit, wobei die Einheit zur Reinigung des CO₂ Gasstroms mindestens einen Einlass für den CO₂ Gasstrom als aufzureinigenden CO₂ Gasstrom aufweist, der in Fluidverbindung mit dem CO₂-Auslass der Einheit zur Bereitstellung des CO₂ Gasstroms steht und wobei die Einheit zur Reinigung des CO₂ Gasstroms mindestens einen Auslass für einen gereinigten CO₂ Gasstrom aufweist; und
mindestens eine Einheit zur Reduktion des mindestens einen bereitgestellten, gegebenenfalls gereinigten, CO₂ Gasstroms zu Kohlenmonoxid, enthaltend mindestens einen Einlass für den CO₂ Gasstrom, der in Fluidverbindung mit mindestens einem Auslass der Einheit zur Bereitstellung eines CO₂ Gasstroms, gegebenenfalls über die mindestens eine Einheit zur Reinigung des CO₂ Gasstroms, steht, und enthaltend mindestens einen Auslass für Kohlenmonoxid;
mindestens eine Einheit zur Herstellung von Phosgen, enthaltend
   mindestens einen Einlass für Chlorgas, der mit einer Quelle für Chlorgas in Fluidverbindung steht, und
   mindestens einen Einlass für Kohlenmonoxid, der in Fluidverbindung mit mindestens einem Auslass für Kohlenmonoxid der Einheit zur Reduktion des besagten CO₂ Gasstroms steht;
   mindestens einen Auslass für Phosgen.

Unter einer Fluidverbindung wird eine Vorrichtung verstanden, die Vorrichtungsteile miteinander verbindet und durch die sich ein Stoff, der in jedem Aggregatzustand vorliegen kann, als Stoffstrom von einem Vorrichtungsteil zum nächsten transportieren lässt, beispielsweise eine Zuleitung in Form eines Rohres. Der Begriff "in Fluidverbindung stehen" bedeutet, dass benannte Vorrichtungsteile über eine Fluidverbindung miteinander verbunden sind.

Ein "Reaktionsraum" ist ein Volumen einer Vorrichtung, in dem die an einer chemischen Reaktion beteiligten Reaktionspartner zusammen vorliegen und in dem die chemische Reaktion stattfindet. Dies kann für eine chemische Reaktion beispielsweise das Volumen eines Gefäßes oder Behälters sein, in dem sich die Reaktionspartner gemeinsam befinden und zur Reaktion gebracht werden sollen.

Eine bevorzugte Ausführungsform der Vorrichtung eignet sich für die gleichzeitige Ausführung der chemolytischen Umsetzung und der Hydrolyse in einem Schritt und ist dadurch gekennzeichnet, dass die Einheit zur Bereitstellung eines CO₂ Gasstroms eine Einheit zur Hydrolyse von mindestens einer organischen Verbindung enthält, enthaltend
mindestens eine Hydrolysevorrichtung, enthaltend mindestens einen Einlass für ein Chemolysereagenz, mindestens einen Einlass für Wasser enthaltende Flüssigkeit, mindestens einen Einlass für Polyurethan-Material und den mindestens einen Auslass für den CO₂ Gasstrom, wobei in einer Reaktionszone der Hydrolysevorrichtung besagter organischer Stoff und besagte Flüssigkeit kontaktiert werden können und der gebildete CO₂ Gasstrom durch den besagten Auslass herausgeführt werden kann.

Eine weitere bevorzugte Ausführungsform der Vorrichtung eignet sich für die getrennte Ausführung der chemolytischen Umsetzung und der Hydrolyse und ist dadurch gekennzeichnet, dass die Einheit zur Bereitstellung eines CO₂ Gasstroms eine Einheit zur Hydrolyse von mindestens einer organischen Verbindung enthält, enthaltend
mindestens eine Chemolysevorrichtung, enthaltend mindestens einen Einlass für eine Chemolysereagenz enthaltende Flüssigkeit, mindestens einen Einlass für Polyurethan-Material und den mindestens einen Auslass für organisch modifiziertes Carbamat, wobei in der Reaktionszone des Reaktors besagter organischer Stoff und besagte Flüssigkeit kontaktiert werden und zu mindestens einer organischen Verbindung ausgewählt aus organisch modifiziertem Carbamat umgesetzt werden kann; und
mindestens eine Hydrolysevorrichtung, enthaltend mindestens einen Einlass für eine wasserhaltige Flüssigkeit, mindestens einen Einlass für organisch modifiziertes Carbamat und den mindestens einen Auslass für den CO₂ Gasstrom, wobei in einer Reaktionszone der Hydrolysevorrichtung besagtes organisch modifiziertes Carbamat und besagte wasserhaltige Flüssigkeit kontaktiert werden können und der gebildete CO₂ Gasstrom durch den besagten Auslass herausgeführt werden kann, wobei der Einlass für organisch modifiziertes Carbamat mit dem Auslass für organisch modifiziertes Carbamat des Reaktors zur Chemolyse in Fluidverbindung steht.

Es ist im Rahmen dieser Ausführungsform weiter bevorzugt, wenn die Einheit zur Hydrolyse von mindestens einer organischen Verbindung zusätzlich mindestens eine Trennvorrichtung enthält, wobei der Auslass für organisch modifiziertes Carbamat der Chemolysevorrichtung in Fluidverbindung mit dem Einlass der Trennvorrichtung steht und die Trennvorrichtung mindestens einen Einlass für organischen Lösungsmittel, das mit dem zuvor eingesetzten Chemolysereagenz nicht vollständig mischbar ist, aufweist, der mit eine Quelle des besagten organischen Lösungsmittels in Fluidverbindung steht und mindestens einen Auslass für eine Carbamatphase enthält, der mit dem Einlass der Hydrolysevorrichtung in Fluidverbindung steht. Die Trennvorrichtung ist derart ausgestaltet, dass die bei der Chemolyse erhaltene Zusammensetzung, zumindest umfassend mindestens ein organisch modifiziertes Carbamat, mit einem organischen Lösungsmittel, das mit dem zuvor eingesetzten Chemolysereagenz nicht vollständig mischbar ist, kontaktiert werden kann und nach Durchmischen und Inkontaktbringen eine Phasentrennung in eine Carbamatphase (enthaltend organisch modifiziertes Carbamat) und eine Lösungsmittelphase bewirkt werden kann, von der die Carbamatphase abgetrennt und aus der Trennvorichtung ausgebracht werden kann. Die Carbamatphase wird im Rahmen dieser Ausführungsform in die Hydrolysevorrichtung eingebracht.

Im Rahmen einer weiteren bevorzugten Ausführungsform der Vorrichtung ist die Einheit zur Reduktion des mindestens einen bereitgestellten, gegebenenfalls gereinigten, CO₂ Gasstroms (31, 31a) eine Elektrolyseeinheit mit Gasdiffusionselektrode zur CO₂ Reduktion oder ein reverse watergas shift Reaktor.

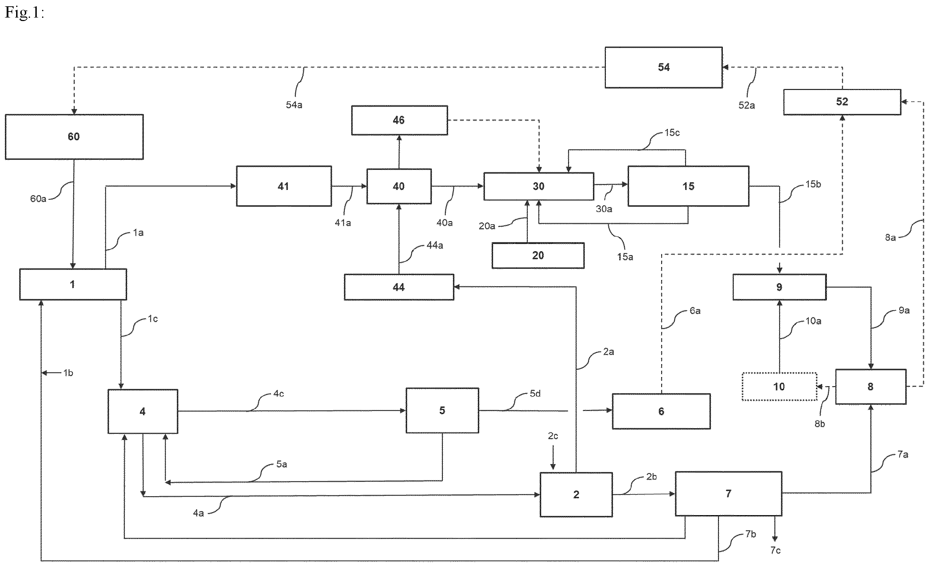

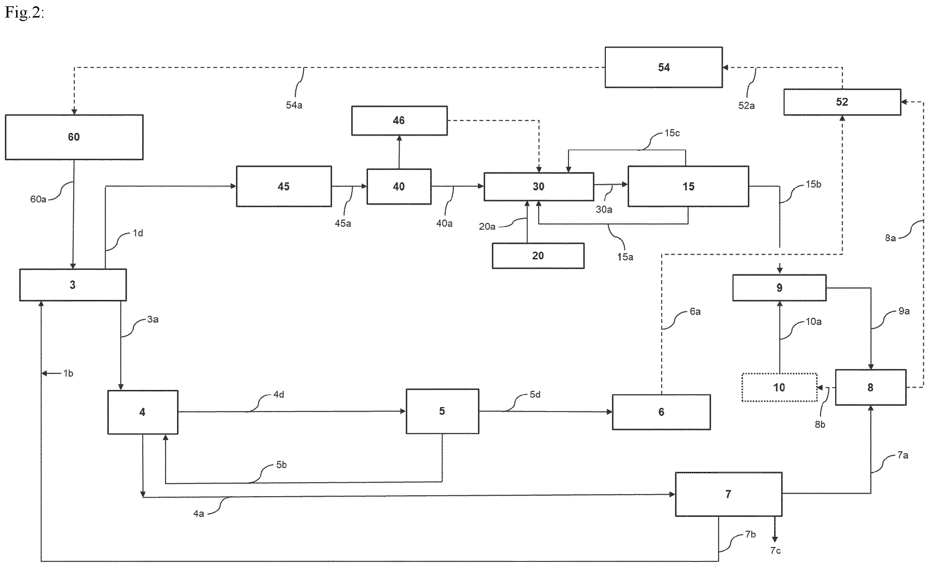

Die Erfindung wird nachstehend anhand der Figuren 1 und 2 beispielhaft durch die Beispiele 1 bis 4 näher erläutert, ohne die Erfindung auf diese Beispiele einzuschränken. In den Figuren 1 und 2 haben die folgenden Bezugszeichen die jeweils rechtsstehende Bedeutung:
1 Chemolyse, hier: Glykolyse
1a Gasstrom CO₂ aus Chemolyse (Glykolyse)
1b Chemolysereagenz (hier: Diethylenglykol (DEG)) Zusatz als Ersatz für Verluste
1c Polyol aus Chemolyse
1d Gasstrom CO₂ aus der gekoppelten Chemolyse mit Hydrolyse (hier: Hydroglykolyse)
1e H₂O + Katalysator Na₂CO₃ für gekoppelte Chemolyse und Hydrolyse (hier: Hydroglykolyse)
2 Hydrolyse
2a Gasstrom CO₂ aus Hydrolyse
2b TDA aus Hydrolyse
2c H₂O zur Hydrolyse
3 gekoppelte Chemolyse mit Hydrolyse (hier: Hydroglykolyse)
3a Polyol Lösungsmittel aus Hydroglykolyse
4 Solvent Extraktion
4a organsiche Aminoverbindung (hier: TDA) / organisch modifizierte Carbamate aus Solvent Extraction
4b H₂O Ersatz Verluste
4c Polyol Toluol aus Solvent Extraction
4d Polyol und Lösungsmittel aus Solvent Extraction
5 Solvent Evaporation
5a Toluol aus Solvent evaporation
5b Lösungsmittel Cyclohexan aus 5
5c Lösungsmittel Ersatz für Verluste
5d Polyol
6 Polyol-Herstellung
6a Polyol aus Polyol-Herstellung
6b Ausschleusung Reste
7 Amin Reinigung
7a organische Aminoverbindung (hier aTDA) aus Amin Reinigung
7b Chemolysereagenz (hier DEG) aus der Amin-Reinigung
7c Rest
7d H₂O aus der Amin Reinigung
7f H₂O aus Amine Purification
8 Isocyanat-Herstellung
8a TDI
8b HCl aus Isocyanatherstellung
9 Phosgen-Herstellung
9a Phosgen
10 HCl Recycling
10a Chlor aus dem HCl Recycling
11 PU-Schaum-Herstellung
15 Prozessgasaufbereitung CO durch: Kühlung, Trocknung, CO₂-Abtrennung (Aminwäsche) & H₂-CO Trennung
15a H₂ aus der H₂-CO Trennung
15b CO aus der H₂-CO Trennung
15c CO₂ aus der CO₂-Abtrennung
20 H₂ Herstellung
20a H₂ aus Wasserelektrolyse
20c H₂O zur Wasserelektrolyse Ersatz Verluste
30 CO Herstellung durch Reduktion von CO₂ (CO₂ Elektrolyse oder RWGS) 30a CO-Produktgasstrom aus der Reduktion von CO₂
40 CO₂ Verdichtung
40a CO₂ nach Verdichtung
41 CO₂ Aufbereitung (CO₂ aus Chemolyse)
41a CO₂ aus Chemolyse nach Aufbereitung
41b CO₂ Purge Strom aus 41
41c CO₂ Purge Strom aus 44
44 CO₂ Aufbereitung aus Hydrolyse
44a CO₂ gereinigt aus Hydrolyse
45 CO₂ Aufbereitung Hydroglykolyse
45a CO₂ gereinigt aus Aufbereitung Hydroglykolyse
45b CO₂ Purge aus Aufbereitung 45
46 CO₂ Lager (optional)
52 Herstellung Polyurethan-Material (hier: PU-Schaum Herstellung)
52a Polyurethanmaterial für den Markt
54 Markt / Wertstoff Sammlung / Trennung
54a Recycelter PU-Schaum
60 Bereitstellung Polyurethan-Material (hier: PU-Schaumvorbereitung & Inertisierung)
60a Polyurethan-Material (hier: TDI basierter PU_Schaum)
70 Heizung RWGS elektrisch oder BioGas oder H₂

### Beispiele

### Beispiel 1 (Fig. 1):

Eine schematische Übersicht über das Gesamtverfahren zur Herstellung von emissionsarmen Toluol-diisocyanat (TDI), CO Herstellung mittels RWGS, deren Beheizung mit Bio-Erdgas erfolgt, HCl Recycling mittels Ch-Herstellung durch thermokatalytische Gasphasenoxidation (Deacon) von HCl und H₂ Bereitstellung aus einer Wasserelektrolyse und CO₂ Bereitstellung aus Chemolyse von Polyurethan-Material (TDI basierte Schäume) mit Diethylenglykol (DEG) als Chemolysereagenz und Hydrolyse der resultierenden organisch modifizierten Carbamate in einem separaten Schritt.

Es werden 300 kg eines Polyurethan-Materials **60a** in Form eines Restfeuchtigkeit enthaltenden Polyurethanschaumes mit einem Gewichtsanteil von 33 Gew.-% TDI und 67 Gew.-% Polyol (ARCOL^{®} Polyol 1108) bereitgestellt. Dieser wird mit 300 kg Diethylenglykol als Chemolysereagenz unter Zusatz von 5,5 kg Na₂CO₃ als Katalysator in einer Chemolyse **1** unter einer Kohlendioxid Inertgasatmosphäre aufgelöst. Das bei der Auflösung erhaltene Produktgemisch mit 227.1 kg Polyol, 251,1 kg Diethylenglykol und 119,6 g TDA/organisch modifizierter Carbamate wird vor der Hydrolyse **2** in eine Solvent Extraction **4** mit Toluol als Lösungsmittel gegeben, die analog Beispiel aus der Druckschrift WO 2020/260387 A1 durchgeführt wird. Die nach der Phasentrennung erhaltene Lösemittelphase mit 1367 kg Toluol und 227.1 kg Polyol wird in eine Solvent Evaporation **5** übergeführt, dort das Lösungsmittel Toluol nahezu vollständig vom Polyol abgetrennt und in die Solvent Extraction **4** zurückgeführt. Das erhaltene Polyol wird in der Polyolherstellung **6** weiter aufgearbeitet.

Die nach der Phasentrennung in der Solvent Extraction **4** erhaltene Carbamatphase enthaltend 119,6 kg TDA/organisch modifiziertes Carbamat wird in die Hydrolyse **2** übergeführt und dort in einer Kohlendioxid Inertgasatmosphäre unter Zugabe von in Summe 16,5 kg Wasser für 2,5 h bei 180°C hydrolysiert. Dabei werden insgesamt eine flüssige Zusammensetzung mit 51,1 kg TDA und 300 kg Diethylenglykol, sowie ein CO₂ Gasstrom **2a** erhalten. Die flüssige Zusammensetzung wird in eine Aminreinigung **7** übergeführt und dort in 47,92 kg TDA **7a** sowie ca. 300 kg Diethylenglykol **7b** und Reststoff **7c** aufgetrennt.

Aus der Chemolyse **1** wird ein Gasstrom **1a** mit 3,7 kg/h CO₂, der Spuren von N₂ und O₂, DEG, Wasser enthält, entnommen und der CO₂-Aufbereitung **41** zugeführt. Der Gasstrom **1a** hat eine Temperatur von 180°C und einen Druck von 1,01 bar_{(abs)}. Der Gasstrom **1a** wird mittels Wärmetauscher auf 25°C abgekühlt und dabei weitgehend von Wasser und DEG befreit.

Anschließend wird der Gasstrom **1a** via Zeolith getrocknet, ein Teilstrom **41a** mit 0,3 kg CO₂ gepurged und der Restgasstrom **41a** mit 3,4 kg CO₂ mit 25°C der Verdichtung **40** zugeführt.

Der Hydrolyse **2** wird ein CO₂ Gasstrom **2a** mit 33,2 kg/h CO₂ sowie Spuren von N₂ und O₂, DEG, Wasser, Toluol entnommen. Der Gasstrom hat eine Temperatur von 180°C und einen Druck von 1,01 bar_{(abs)}. Der Gasstrom **2a** wird mittels Wärmetauscher auf 25°C abgekühlt und dabei weitgehend von Wasser und DEG befreit.

Anschließend wird der abgekühlte Gasstrom via Zeolith getrocknet, ein Teilstrom mit 2 kg/h CO₂ gepurged und der Restgasstrom **44a** mit 31,2 kg/h CO₂ mit 25°C der Verdichtung **40** zugeführt.

Der Verdichterstufe **40** wird ein kontinuierlicher Gasstrom **40a** mit 34,6 kg/h CO₂ mit 30 bar und 40°C entnommen und einer CO Herstellung durch Reduktion von CO₂ **30** in Form einer RWGS in den Reaktionsraum der RWGS zugeführt.

Die RWGS Reaktion wird bei 802°C betrieben, wobei zur Aufrechterhaltung der Reaktionstemperatur Bio-Erdgas einspeist und verbrannt wird. Optional könnte auch H₂ zum Heizen eingesetzt oder elektrisch geheizt werden. In den RWGS Reaktionsraum der CO Herstellung durch Reduktion von CO₂ **30,** der bei einer Temperatur von 802°C und 30 bar betrieben wird, werden 34,6 kg/h CO₂ sowie 1,57 kg/ h H₂ **20a** aus einer Wasserelektrolyse **20** eingebracht.

Aus der RWGS Reaktion wird das erhaltene Produktgasgemisch **30a** bestehend aus CO, H₂O, nicht umgesetztem CO₂ sowie nicht umgesetzter H₂ sowie Nebenprodukten, hauptsächlich geringe Mengen an Methan, entnommen und in einer Prozessgaskühlung der Prozessgasaufbereitung CO **15** abgekühlt.

Danach wir die Gasmischung einer Prozessgastrocknung der Prozessgasaufbereitung CO **15** zugeführt, bei der in Summe ca. 14,1 kg/h Wasser abgetrennt werden. Dieses Wasser wird der Wasserelektrolyse **20** zugeführt.

Aus der Wasserelektrolyse **20** werden insgesamt 1,57 kg/h Wasserstoff entnommen und es werden 14,13 kg/h Wasser der Wasserelektrolyse zugeführt. Dieses Wasser setzt sich zusammen aus dem zurückgeführten Wasser aus der Prozessgastrocknung sowie frisch zugesetztem Wasser.

Das verbleibende Gasgemisch aus der Prozessgastrocknung wird einer CO₂ Abtrennung der Prozessgasaufbereitung CO **15** zugeführt. Die CO₂ Abtrennung erfolgt mittels Aminwäsche, wobei das abgetrennte CO₂ **15c** wieder der Reduktion von CO₂ **30** (RWGS Reaktion) zugeführt wird.

Die Energie zur CO₂ Abtrennung aus dem gebildeten CO₂-Aminkomplex wird aus der o.g. Prozessgaskühlung, in der die RWGS Reaktionsgase Gase abgekühlt werden, bezogen. Das von CO₂ befreite Gas wird der H₂-CO Trennung Abtrennung der Prozessgasaufbereitung CO **15** zugeführt. Für die H₂-CO Trennung wird eine sogenannte Cold-box eingesetzt, bei der das H₂-CO Gasgemisch abgekühlt und Wasserstoff und CO getrennt werden. Der abgetrennte Wasserstoff (15a) wird der RWGS Reaktion (31) wieder zugeführt. Aus der H₂-CO Trennung werden 22,0 kg/h CO **15b** einer Phosgensynthese **9** zugeführt. Hierbei reagierte das CO **15b** mit 55,78 kg/h Chlor **10a**, welches aus einem HCl-Recycling Prozess **10** entnommen wurde. Das HCl Gas wird durch thermokatalytische Gasphasenoxidation (Deacon) von HCl aus der Isocyanat-Herstellung **8** zu Chlor umgesetzt. Optional kann der O₂ aus der Wasserelektrolyse **20** für die Oxidation von HCl-Gas im Deacon Prozess **10** eingesetzt werden.

Aus der Phosgensysnthese **9** werden 77,78 kg/h Phosgen entnommen und in einer Isocyanat-Herstellung **8** mit 47,92 kg/h Toluol-diamin **7a** zu 68,35 kg/h Toluol-diisocyanat **8a** umgesetzt.

Das dabei anfallende HCl-Gas mit einer Menge von 57,35 kg/h wird nach Reinigung über eine Tieftemperaturdestillation einer Ch-Herstellung durch thermokatalytische Gasphasenoxidation **10** zugeführt. In der thermokatalytische Gasphasenoxidation **10** wird das HCl-Gas bei ca. 300°C an einem Rutheniumoxid-basierten Katalysator mit Sauerstoff zu Chlor und H₂O umgesetzt. Der benötigte Sauerstoff von 12,57 kg/h wird aus der Wasserelektrolyse **20** entnommen.

Das erhaltene Toluol-diisocyanat 68,35 kg/h **8a** wird mit dem aus dem Prozess erhaltenen 227,1 kg/h Polyol (entspricht im Wesentlichen der Spezifikation von ARCOL^{®} Polyol 1108) **6a** wieder zu 295,44 kg/h Polyurethan-Material in einer PU-Schaum-Herstellung **52** umgesetzt.

Nach Nutzung des Polyurethan-Materials in diversen Anwendungen im Markt **54,** kann dieses gesammelt und recycelt werden, um den daraus erhaltenen Polyurethan-Material als Wertstoff nach Aufbereitung und Vorbereitung **60** der Chemolyse (1) bereitzustellen.

Der Wasserstoff **20a** wird in einer Wasserelektrolyse **20** mit einer Leistung von 0,088 MW erzeugt, wobei regenerative Energie eingesetzt wird. Die Wasserelektrolyse **20** ist eine alkalische Wasserelektrolyse, die mit einer Stromdichte von 8 kA/m² und einer Zellspannung von 2 V je Elektrolyseelement betrieben wird. Dabei werden 0,088 MW und 14,13 kg/h Wasser (16a+20c) aus der Prozessgaskühlung , der Prozessgas-Trocknung jeweils aus der Abtrennung der Prozessgasaufbereitung CO **15** sowie extern zugeführt. Der Wasserelektrolyse werden 1,57 kg/h H₂ und 12,56 kg/h Sauerstoff entnommen.

Durch das erfindungsgemäße Verfahren werden nahezu vollständig alles Rohstoffe des TDI basierten PU-Materials wie das Amin und das Polyol recycelt und damit der Wertschöpfungskreis geschlossen.

Durch Einsatz von regenerativer Energie bei der Wasserelektrolyse wird der CO₂-Footprint des aus CO und Cl₂ hergestellten Phosgens weiterhin erniedrigt und ermöglicht so ein nachhaltiger hergestelltes TDI bzw. das daraus resultierenden PU-Material.

### Beispiel 2 (Fig 1):

Dieses Beispiel entspricht abgesehen von der Ausführung der CO₂-Reduktion dem Beispiel 1.

Als Reduktion von CO₂ **30** wird eine Niedertemperatur COz-Elektrolyse durchgeführt. Die CO₂-Elektrolyse wird dabei zweckmäßigerweise gemäß der europäischen Patentanmeldung Anmeldenummer 18195279.7, Beispiel 1 betrieben. Die Elektrolyse wird bei einem Druck von 1,2 bar_{(abs)} betrieben. Es werden 10 Elemente von je 1,6 m² Elektrodenfläche eingesetzt, die zu einem Elektrolyseur zusammen geschaltet werden. Die Elektrolyse wird bei einer Zellspannung von 3,7 V, einer Stromdichte von 4,05 kA/m² mit einer Stromausbeute bzgl. CO von 65% betrieben. Es werden 0,239 MWh an regenerativ erzeugter Energie, insbesondere Windstrom, verbraucht, entsprechend einer Anschlussleistung von 0,239 MW.

34,6 kg/h CO₂ werden der COz-Elektrolyse **30** zugeführt nach der Trocknung, Reinigung in **41** und Verdichtung **40** mit einem Druck von 1,2 bar_{(abs)} zugeführt. Aus der COz-Elektrolyse **30** wird eine Gasmischung **30a** von 22 kg/h CO, 200 kg/h CO₂, 0,85 kg/h H₂ entnommen.

Weiterhin werden dem Anodenraum der Elektrolyse 19,36 kg/h O₂ entnommen.

Das aus der Elektrolyse **30** hergestellte Gasgemisch **30a** wird einer Prozessgasaufbereitung **15** mit einer Trocknung & einer CO₂-Abtrennung in Form einer Aminwäsche zugeführt und das nicht umgesetzte CO₂ **15c** aus dem Gemisch abgetrennt und in die Elektrolyse **30** zurückgeführt. Das von CO₂ befreite Gas bestehend aus CO und H₂ wird einer CO-H₂ Trennung in Form einer Cold-box zugeführt, in der die Trennung von CO und H₂ erfolgt. 22 kg/h CO **15b** wird mit dem aus dem HCl-Recycling **10** stammende Cl₂ **10a** zu Phosgen **9a** in der Phosgen-Herstellung **9** umgesetzt und dieses mit dem aus der Hydrolyse **2** und der Amin-Reinigung 7 stammenden TDA **7a** zu TDI **8a** in der Isocyanat-Herstellung **8** umgesetzt.

### Beispiel 3 (Fig 2):

Fig. 2 Eine schematische Übersicht über das Gesamtverfahren zur Herstellung von emissionsarmen Toluol-diisocyanat (TDI), mit rWGS Reaktion, Chlorherstellung, PU Herstellung, Verwendung und Verwertung des Polyurethan-Material Abfalls und das Recycling via Hydroglykolyse und Nutzung des CO₂ für die RWGS Reaktion

Es werden 300 kg eines Polyurethan-Materials **60a** in Form eines Restfeuchtigkeit enthaltenden Polyurethanschaumes mit einem Gewichtsanteil von 33 Gew.-% TDI und 67 Gew.-% Polyol (ARCOL^{®} Polyol 1108) bereitgestellt. Dieser wird mit 300 kg Diethylenglykol als Chemolysereagenz unter Zusatz von 5,5 kg Na₂CO₃ als Katalysator in einer kombinierten Chemolyse und Hydrolyse (hier: Hydroglykolyse) **3** mit insgesamt 16,5 kg Wasser unter Kohlendioxid Inertgasatmosphäre wie in Beispiel x der Druckschrift WO XXX beschrieben analog dazu umgesetzt. Es wird eine Produktlösung **3a** und ein CO₂ Gasstrom **1d** erhalten

Die Produktlösung wird nach der Umsetzung in eine Solvent Extraction **4** übergeführt und mit 1866 kg Cyclohexan versetzt. Die nach der Phasentrennung erhaltene Lösemittelphase **4d** mit 1866 kg Cyclohexan, 223.6 kg Polyol und 1.8 kg Reststoff wird in eine Solvent Evaporation **5** übergeführt, dort das Lösungsmittel Cyclohexan nahezu vollständig vom Polyol abgetrennt und in die Solvent Extraction **4** zurückgeführt. Das erhaltene Polyol wird in der Polyolherstellung **6** weiter aufgearbeitet und von den Reststoffen **6b** getrennt.

Die nach der Phasentrennung in der Solvent Extraction **4** erhaltene Amin-haltige Phase (TDA) enthaltend 52 kg TDA Wasser, salze sowie 300 kg DEG in eine Aminreinigung **7** übergeführt und dort in 47,92 kg TDA **7a** sowie ca. 300 kg Diethylenglykol **7b** und Reststoff **7c** aufgetrennt.

Aus der Hydroglykolyse 3 wird ein Gasstrom **1d** mit 37,4 kg/h CO₂, der Spuren von N₂ und O₂, DEG, Wasser enthält entnommen und der CO₂-Aufbereitung **45** zugeführt. Der Gasstrom **1d** hat eine Temperatur von 180°C und einen Druck von 1,01 bar_{(abs)} Der Gasstrom **1d** wird mittels Wärmetauscher auf 25°C abgekühlt und dabei weitgehend von Wasser und DEG befreit. Anschließend wird der Gasstrom **1d** via Zeolith getrocknet, ein Teilstrom **45b** mit 2,8 kg CO₂ gepurged und der Restgasstrom **45a** mit 34,6 kg/h CO₂ mit 25°C der Verdichtung **40** zugeführt.

Der Verdichterstufe **40** wird ein kontinuierlicher Gasstrom **40a** mit 34,6 kg/h CO₂ mit 30 bar und 40°C entnommen und einer CO Herstellung durch Reduktion von CO₂ **30** in Form einer RWGS in den Reaktionsraum der RWGS zugeführt.

Die RWGS Reaktion **30** wird bei 802°C betrieben, wobei zur Aufrechterhaltung der Reaktionstemperatur Bio-Erdgas einspeist und verbrannt wird. Optional könnte auch H₂ zum Heizen eingesetzt oder elektrisch geheizt werden. In den Reaktionsraum der RWGS **30,** der bei einer Temperatur von 802°C und 30bar betrieben wird, werden 34,6 kg/h CO₂ **40a** sowie 1,57 kg/ h H₂ **20a** aus einer Wasserelektrolyse **20** eingebracht.

Aus der RWGS Reaktion wird das erhaltene Produktgasgemisch **30a** bestehend aus CO, H₂O, nicht umgesetztem CO₂ sowie nicht umgesetzter H₂ sowie Nebenprodukten, hauptsächlich geringe Mengen an Methan, entnommen und in einer Prozessgaskühlung der Prozessgasaufbereitung **15** abgekühlt.

Danach wir die Gasmischung einer Prozessgastrocknung Prozessgasaufbereitung **15** zugeführt, bei der in Summe ca.14,1 kg/h Wasser abgetrennt werden. Dieses Wasser wird der Wasserelektrolyse **20** zugeführt.

Aus der Wasserelektrolyse **20** werden insgesamt 1,57 kg/h Wasserstoff **20a** entnommen und es werden 14,13 kg/h Wasser der Wasserelektrolyse zugeführt. Dieses Wasser setzt sich zusammen aus dem zurückgeführten Wasser aus der oben genannten Prozessgastrocknung sowie frisch zugesetztem Wasser.

Das verbleibende Gasgemisch aus der Prozessgastrocknung wird einer CO₂ Abtrennung Prozessgasaufbereitung **15** zugeführt. Die CO₂ Abtrennung erfolgt mittels Aminwäsche, wobei das abgetrennte CO₂ **15c** wieder der RWGS Reaktion **30** zugeführt wird.

Die Energie zur CO₂ Abtrennung aus dem gebildeten CO₂-Aminkomplex wird aus der Prozessgaskühlung der Prozessgasaufbereitung **15,** in der die RWGS Reaktionsgase Gase **30a** abgekühlt werden, bezogen. Das von CO₂ befreite Gas wird der H₂-CO Trennung der Prozessgasaufbereitung **15** zugeführt. Für die H₂-CO Trennung wird eine sogenannte Cold-box eingesetzt, bei der das H₂-CO Gasgemisch abgekühlt und Wasserstoff und CO getrennt werden. Der abgetrennte Wasserstoff **15a** wird der RWGS Reaktion **30** wieder zugeführt. Aus der H₂-CO Trennung werden 22,0 kg/h CO **15b** einer Phosgensynthese **9** zugeführt. Hierbei reagierte das CO **15b** mit 55,78 kg/h Chlor **10a,** welches aus einem HCl-Recycling Prozess **10** entnommen wurde. Das HCl Gas wird durch thermokatalytische Gasphasenoxidation (Deacon) von HCl aus der Isocyanat-Herstellung **8** zu Chlor umgesetzt. Optional kann der O₂ aus der Wasserelektrolyse **20** für die Oxidation von HCl-Gas im Deacon Prozess **10** eingesetzt werden.

Aus der Phosgensysnthese **9** werden 77,78 kg/h Phosgen entnommen und in einer Isocyanat-Herstellung **8** mit 47,92 kg/h Toluol-diamin **7a** zu 68,35 kg/h Toluol-diisocyanat **8a** umgesetzt.

Das dabei anfallende HCl-Gas mit einer Menge von 57,35 kg/h wird nach Reinigung über eine Tieftemperaturdestillation einer Ch-Herstellung durch thermokatalytische Gasphasenoxidation (10) zugeführt. In der thermokatalytische Gasphasenoxidation **10** wird das HCl-Gas bei ca. 300°C an einem Rutheniumoxid-basierten Katalysator mit Sauerstoff zu Chlor und H₂O umgesetzt. Der benötigte Sauerstoff von 12,57 kg/h wird aus der Wasserelektrolyse **20** entnommen.

Das erhaltene Toluol-diisocyanat 68,35 kg/h **8a** wird mit dem aus dem Prozess erhaltenen 223,6 kg/h Polyol (entspricht im Wesentlichen der Spezifikation von ARCOL^{®} Polyol 1108) **6a** wieder zu 291,95 kg/h Polyurethan-Material in einer PU-Schaum-Herstellung **52** umgesetzt.

Nach Nutzung des Polyurethan-Materials in diversen Anwendungen im Markt **54,** kann dieses gesammelt und recycelt werden, um den daraus erhaltenen Polyurethan-Material Abfall nach Aufbereitung und Vorbereitung **60** der Hydroglykolyse **3** bereitzustellen.

Der Wasserstoff **20a** wird in einer Wasserelektrolyse **20** mit einer Leistung von 0,088 MW erzeugt, wobei regenerative Energie eingesetzt wird. Die Wasserelektrolyse **20** ist eine alkalische Wasserelektrolyse, die mit einer Stromdichte von 8 kA/m² und einer Zellspannung von 2 V je Elektrolyseelement betrieben wird. Dabei werden 0,088 MW und 14,13 kg/h Wasser aus der Prozessgaskühlung der Prozessgasaufbereitung (PGA) **15,** der Prozessgas-Trocknung der PGA **15** sowie extern zugeführt. Der Wasserelektrolyse werden 1,57 kg/h H₂ und 12,56 kg/h Sauerstoff entnommen.

Durch das erfindungsgemäße Verfahren werden nahezu vollständig alles Rohstoffe des TDI basierten PU-Materials wie das Amin und das Polyol recycelt und damit der Wertschöpfungskreis geschlossen.

Durch Einsatz von regenerativer Energie bei der Wasserelektrolyse wird der CO₂-Footprint des aus CO und Cl₂ hergestellten Phosgens weiterhin erniedrigt und ermöglicht so ein nachhaltige hergestelltes TDI bzw. das daraus resultierenden PU-Material.

### Beispiel 4 (Fig.2):

Dieses Beispiel entspricht abgesehen von der Ausführung der CO₂-Reduktion dem Beispiel 3.

Als Reduktion von CO₂ **30** wird eine Niedertemperatur COz-Elektrolyse wie in Beispiel 2 beschrieben durchgeführt.

## Patentansprüche

1. Verfahren zur Herstellung von Phosgen durch zumindest folgende Schritte
Herstellung eines CO₂ Gasstroms (31) durch zumindest die folgenden Schritte:
Bereitstellung mindestens eines organisch modifizierten Carbamats;
Hydrolyse des bereitgestellten mindestens einen organisch modifizierten Carbamats, zumindest unter Bildung von organischer Aminoverbindung und CO₂;
Abtrennung des gebildeten CO₂ unter Erhalt mindestens eines CO₂ Gasstroms;
bevorzugt Reinigung (4) des CO₂ Gasstroms (31) von Nebenbestandteilen, insbesondere von Nebenbestandteilen ausgewählt aus mindestens einer Verbindung aus der Liste, die gebildet wird aus Alkoholen, Polyethern, Kohlenwasserstoffen, organischen Aminen, Wasser, Schwefelverbindungen, Staub, Sauerstoff und Stickstoff, mittels mindestens einer Reinigungsmethode, ausgewählt aus Kondensation, Adsorption katalytischer Gasreinigung oder Gaswäsche unter Erhalt eines gereinigten Kohlendioxids (31a);
Reduktion (6) von CO₂ des mindestens einen hergestellten, gegebenenfalls gereinigten CO₂ Gasstroms (31, 31a) zu Kohlenmonoxid (21a);
Synthese (1) von Phosgen (20) zumindest aus dem durch besagte Reduktion erhaltenen Kohlenmonoxid (21a) und Chlor (22).

2. Verfahren gemäß Anspruch 1, **dadurch gekennzeichnet, dass** als bereitgestelltes organisch modifiziertes Carbamat mindestens ein unmittelbares Verfahrensprodukt einer Chemolyse von Polyurethan-Material eingesetzt wird, umfassend mindestens folgende Verfahrensschritte:
Bereitstellung von Polyurethan-Material;
Umsetzung des bereitgestellten Polyurethan-Materials mit mindestens einem Chemolysereagenz ausgewählt aus (a) einem primären oder sekundären organischen Amin, (b) einem Aminoalkohol mit einer primären oder sekundären Aminogruppe oder (c) einem Alkohol mit mindestens einer Hydroxylgruppe,
unter Bildung von mindestens einem organisch modifizierten Carbamat.

3. Verfahren gemäß einem der Ansprüche 1 oder 2, **dadurch gekennzeichnet, dass** der besagte CO₂ Gasstrom (31) durch Chemolyse von Polyurethan-Material hergestellt wird, umfassend mindestens die Schritte
Bereitstellung von Polyurethan-Material;
Umsetzung des bereitgestellten Polyurethan-Materials mit mindestens einem Chemolysereagenz ausgewählt aus (a) einem primären oder sekundären organischen Amin, (b) einem Aminoalkohol mit einer primären oder sekundären Aminogruppe oder (c) einem Alkohol mit mindestens einer Hydroxylgruppe, unter Bildung von mindestens einem organisch modifiziertem Carbamat; Hydrolyse des zuvor gebildeten organisch modifiziertem Carbamats unter Bildung zumindest von organischer Aminoverbindung und CO₂;
Abtrennung des gebildeten CO₂ unter Erhalt mindestens eines CO₂ Gasstroms.

4. Verfahren gemäß Anspruch 3, **dadurch gekennzeichnet, dass** die besagte Umsetzung des bereitgestellten Polyurethan-Materials zu besagtem organisch modifiziertem Carbamat und die besagte Hydrolyse des besagten Carbamats gemeinsam in einem Verfahrensschritt verwirklicht werden.

5. Verfahren gemäß einem der Ansprüche 2 bis 4, **dadurch gekennzeichnet, dass** der erhaltene CO₂ Gasstrom eine Temperatur von 140 °C bis 220 °C, bevorzugt von 170 °C bis 200 °C, aufweist.

6. Verfahren gemäß einem der Ansprüche 2 bis 5, **dadurch gekennzeichnet, dass** die besagte Umsetzung des bereitgestellten Polyurethan-Materials zu besagtem organisch modifiziertem Carbamat unter Einsatz mindestens eines Katalysators erfolgt, insbesondere ausgewählt aus Carbonat, Hydrogencarbonat, Orthophosphat, Mono-Hydrogen-Orthophosphat, Metaphosphat, Hydroxid, organisches Amin, organometallische Verbindungen oder Mischungen von zwei oder mehr der vorgenannten Katalysatoren.

7. Verfahren gemäß einem der Ansprüche 2 bis 6, **dadurch gekennzeichnet, dass** das Chemolysereagenz ausgewählt wird aus Ethylenglykol, Diethylenglykol, Propylenglykol, Dipropylenglykol, Methylglykol, Triethylenglykol, Glycerin, 2 Methyl-1,3-Propandiol oder Mischungen von zwei oder mehr der vorgenannten Alkohole.

8. Verfahren gemäß einem der vorangehenden Ansprüche, **dadurch gekennzeichnet, dass** eine Reinigung (4) des CO₂ Gasstroms (31) von Nebenbestandteilen erfolgt und hierbei zumindest der CO₂ Gasstrom über mehrere Kondensatoren geführt und abgekühlt wird, wobei die im Gasstrom als Nebenbestandteil enthaltenen organischen und wässrigen Komponenten kondensieren, als Flüssigkeit abgetrennt werden und nach Abschluss der Reinigung (4) ein gereinigter CO₂ Gasstrom (31a) der Reduktion (6) zu Kohlenstoffmonoxid zugeführt wird.

9. Verfahren gemäß einem der vorangehenden Ansprüche, **dadurch gekennzeichnet, dass** ein Wasserstoffstrom (29a) bereitgestellt wird und zusammen mit dem gegebenenfalls gereinigten CO₂ Gasstrom (31, 31a) nach dem Prinzip der reverse water gas shift-Reaktion zu einem Produktgas (39), enthaltend Kohlenstoffmonoxid (21) und gegebenenfalls Nebenprodukte (32) umgesetzt wird.

10. Verfahren gemäß Anspruch 9, **dadurch gekennzeichnet, dass** zur Bereitstellung des Wasserstoffstroms (29a) eine Elektrolyse (5) von Wasser (26) zu Wasserstoff (29) und Sauerstoff (27) ausgeführt wird.

11. Verfahren gemäß Anspruch 10, **dadurch gekennzeichnet, dass** die Wasserelektrolyse (5) unter Verwendung von aus regenerativer Energie erzeugtem elektrischem Strom durchgeführt wird, insbesondere aus regenerativer Energie in Form von Windkraft, Sonnenenergie oder Wasserkraft.

12. Verfahren gemäß einem der vorangehenden Ansprüche, **dadurch gekennzeichnet, dass** der gegebenenfalls gereinigte CO₂ Gasstrom für die Reduktion (6) in eine Elektrolysevorrichtung eingebracht wird und an einer Elektrode, bevorzugt an einer Gasdiffusionselektrode, zu Kohlenstoffmonoxid reduziert wird.

13. Vorrichtung zur Herstellung von Phosgen, enthaltend
mindestens eine Einheit zur Bereitstellung eines CO₂ Gasstroms, der unmittelbares Produkt eines Verfahrens ist, das zumindest den Schritt einer Hydrolyse mindestens einer organischen Verbindung ausgewählt aus organisch modifiziertem Carbamat enthält, wobei diese Einheit zumindest mindestens einen CO₂-Auslass für besagten CO₂ Gasstrom umfasst;
gegebenenfalls mindestens eine Einheit zur Reinigung des CO₂ Gasstroms (31) von Nebenbestandteilenenthaltend mindestens eine Einheit aus der Gruppe, die gebildet wird aus Kondensationseinheit, Adsorptionseinheit, Gaswäscheeinheit oder katalytischer Gasreinigungseinheit, wobei die Einheit zur Reinigung des CO₂ Gasstroms mindestens einen Einlass für den CO₂ Gasstrom (31) als aufzureinigenden CO₂ Gasstrom aufweist, der in Fluidverbindung mit dem CO₂-Auslass der Einheit zur Bereitstellung des CO₂ Gasstroms steht und wobei die Einheit zur Reinigung des CO₂ Gasstroms mindestens einen Auslass für einen gereinigten CO₂ Gasstrom aufweist;
mindestens eine Einheit zur Reduktion des mindestens einen bereitgestellten, gegebenenfalls gereinigten, CO₂ Gasstroms (31, 31a) zu Kohlenmonoxid (21a), enthaltend mindestens einen Einlass für den CO₂ Gasstrom, der in Fluidverbindung mit mindestens einem Auslass der Einheit zur Bereitstellung eines CO₂ Gasstroms, gegebenenfalls über die mindestens eine Einheit zur Reinigung des CO₂ Gasstroms, steht, und enthaltend mindestens einen Auslass für Kohlenmonoxid;
mindestens eine Einheit zur Herstellung von Phosgen, enthaltend
mindestens einen Einlass für Chlorgas, der mit einer Quelle für Chlorgas in Fluidverbindung steht, und
mindestens einen Einlass für Kohlenmonoxid, der in Fluidverbindung mit mindestens einem Auslass für Kohlenmonoxid der Einheit zur Reduktion des besagten CO₂ Gasstroms steht;
mindestens einen Auslass für Phosgen.

14. Vorrichtung nach Anspruch 13, **dadurch gekennzeichnet, dass** die Einheit zur Bereitstellung eines CO₂ Gasstroms eine Einheit zur Hydrolyse von mindestens einer organischen Verbindung enthält, enthaltend
mindestens eine Hydrolysevorrichtung, enthaltend mindestens einen Einlass für eine Wasser und Chemolysereagenz enthaltende Flüssigkeit, mindestens einen Einlass für Polyurethan-Material und den mindestens einen Auslass für den CO₂ Gasstrom, wobei in einer Reaktionszone der Hydrolysevorrichtung Polyurethan-Material und besagte Flüssigkeit kontaktiert werden können und der gebildete CO₂ Gasstrom durch den besagten Auslass herausgeführt werden kann.

15. Vorrichtung nach Anspruch 14, **dadurch gekennzeichnet, dass** die Einheit zur Bereitstellung eines CO₂ Gasstroms eine Einheit zur Hydrolyse von mindestens einer organischen Verbindung enthält, enthaltend
mindestens eine Chemolysevorrichtung, enthaltend mindestens einen Einlass für eine Chemolysereagenz enthaltende Flüssigkeit, mindestens einen Einlass für Polyurethan-Material und den mindestens einen Auslass für organisch modifiziertes Carbamat, wobei in der Reaktionszone des Reaktors Polyurethanmaterial und besagte Flüssigkeit kontaktiert werden und zu mindestens einer organischen Verbindung ausgewählt aus organisch modifiziertem Carbamat umgesetzt werden kann;
mindestens eine Hydrolysevorrichtung, enthaltend mindestens einen Einlass für ein Chemolysereagenz, mindestens einen Einlass für Wasser enthaltende Flüssigkeit, organisch modifiziertes Carbamat und den mindestens einen Auslass für den CO₂ Gasstrom, wobei in einer Reaktionszone der Hydrolysevorrichtung besagtes organisch modifiziertes Carbamat und besagte wasserhaltige Flüssigkeit kontaktiert werden können und der gebildete CO₂ Gasstrom durch den besagten Auslass herausgeführt werden kann, wobei der Einlass für organisch modifiziertes Carbamat mit dem Auslass für organisch modifiziertes Carbamat der Chemolysevorrichtung in Fluidverbindung steht.

16. Vorrichtung nach einem der Ansprüche 13 bis 15, **dadurch gekennzeichnet, dass** die Einheit zur Reduktion des mindestens einen bereitgestellten, gegebenenfalls gereinigten, CO₂ Gasstroms (31, 31a) eine Elektrolyseeinheit mit Gasdiffusionselektrode zur CO₂ Reduktion oder ein reverse watergas shift Reaktor ist.
